# EUROPÄISCHE PATENTSCHRIFT

(11) **EP 3 116 563 B1**
(45) Veröffentlichungstag und Bekanntmachung des Hinweises auf die Patenterteilung: **16.01.2019**
(21) Anmeldenummer: 15709507.6
(22) Anmeldetag: 13.03.2015
(51) Int. Cl.: A61M 1/16

(54) **FLÜSSIGKEITSKASSETTE MIT KIPPTOLERANTER ZENTRIERVERRASTUNG SOWIE BLUTBEHANDLUNGSVORRICHTUNG**
FLUID CASSETTE HAVING A TILT-TOLERANT CENTERING LOCKING, AND BLOOD TREATMENT DEVICE
CASSETTE DE LIQUIDE COMPORTANT UN CRAN D'ARRÊT DE CENTRAGE RÉSISTANT AU BASCULEMENT, ET DISPOSITIF DE TRAITEMENT SANGUIN

(30) Priorität: 14.03.2014 DE 102014103492
(43) Veröffentlichungstag der Anmeldung: 18.01.2017
(73) Patentinhaber: Fresenius Medical Care Deutschland GmbH, 61352 Bad Homburg (DE)
(72) Erfinder: LAUER, Martin, 66606 St. Wendel (DE)
(74) Vertreter: Bobbert & Partner Patentanwälte PartmbB
(86) Internationale Anmeldenummer: PCT/EP2015/055299
(87) Internationale Veröffentlichungsnummer: WO 2015/136077

(56) Entgegenhaltungen:
- EP-A2- 0 223 580
- DE-A1-102009 012 632
- DE-A1-102009 012 633
- DE-A1-102010 032 181
- US-A1- 2013 079 635
- US-A1- 2013 248 629

## Beschreibung

Die vorliegende Erfindung betrifft eine Flüssigkeitskassette gemäß dem Oberbegriff des Anspruchs 1 und eine Blutbehandlungsvorrichtung gemäß dem Oberbegriff des Anspruchs 10.

Einmalteilsysteme werden in der Medizin- oder Labortechnik zunehmend als kompakte medizinische Funktionsvorrichtungen wie Kassettensysteme oder Blutbehandlungskassetten ausgeführt, in denen Flüssigkeiten und Gase, insbesondere medizinische Flüssigkeiten und Blut, in Kanälen und Kammern geführt werden. Sind sie zum einmaligen Gebrauch vorgesehen, so spricht man von Kassettendisposables oder Einwegkassetten.

Zumeist handelt es sich hierbei um Hartteil-Folie-Kassetten. Das Hartteil besteht regelmäßig aus einem Spritzgußwerkstoff wie beispielweise PE, PP, PA, ABS, PMMA, PC oder PVC. In ihm sind beispielsweise Schlauchanschlüsse, Konnektoren, Kammern, Kanäle und Zentriereinrichtungen ausgestaltet. Zu ihrer Verwendung wird die Blutbehandlungskassette beim Einsetzen in die Blutbehandlungsvorrichtung zwischen eine Tür und eine Aktor-Sensor-Einheit der Blutbehandlungsvorrichtung eingesetzt und anschließend die Tür durch ihr Schließen in eine sog. Verpress-Stellung gebracht, bei der die Folie gegen das Hartteil gepresst und die Blutbehandlungskassette mit der Folie räumlich definiert an die Aktor-Sensor-Matte der Aktor-Sensor-Einheit angekoppelt wird. Beim Einsetzen der Blutbehandlungskassette - auch als deren Aufrüsten bezeichnet - wird die Haupterstreckungsebene der Blutbehandlungskassette gegen die Haupterstreckungsebene der Blutbehandlungsvorrichtung (meist die Aktor-Sensor-Matte) ausgerichtet und verpresst. In die Aktor-Sensor-Matte und Aktor-Sensor-Platte (oder -einheit) integrierte Aktoren (hierin auch als Aktuatoren bezeichnet; Komponenten, die der Aktor-Sensor-Platte oder -Matte zuzuordnen sind, werden hierin auch als als "aktor-sensor-seitig" bezeichnet) können Bewegungen durch die Folie hindurch ausüben, wodurch beispielsweise Pump- oder Ventil-Funktionen realisierbar sind. Mittels wenigstens eines, optional in der Aktor-Sensor-Platte vorgesehenen, Sensors können beispielsweise Eigenschaften von Fluiden, welche die Blutbehandlungskassette durchströmen, gemessen werden.
Aus der US 2013/0248629 A1 sind Vorrichtungen zur Versorgung mit medizinischen Flüssigkeiten bekannt.

Vorrichtungen zum Verbinden einer externen Funktionseinrichtung mit einer Anordnung und Verfahren zum Verbinden sind aus der DE 10 2009 012 633 A1 bekannt.

Eine Abdichtungseinrichtung zum Abdichten eines Volumens einer medizinischen Behandlungsanordnung gegen ein weiteres Volumen sowie Anordnung und Verfahren sind aus der DE 2009 012 632 A1 bekannt.

Ankoppeleinrichtung, Konnektor, medizinische Funktionseinrichtung und Behandlungsvorrichtung sind in der DE 10 2010 032 181 A1 offenbart.

In der EP 0 223 580 A2 ist eine Membranpumpe offenbart.

Verfahren zur bildgesteuerten Überwachung der Abgabe von Flüssigkeiten in den menschlichen Körper sind aus der US 2013/0079635 A1 bekannt.

Eine erfindungsgemäße Aufgabe kann darin bestehen, eine weitere medizinische Flüssigkeitskassette (Kassette zur Aufnahme medizinischer Flüssigkeiten), insbesondere eine Blutbehandlungskassette, mit einer Vorrichtung zum Zentrieren der medizinischen Flüssigkeitskassette (im Folgenden kurz: Flüssigkeitskassette) an der Blutbehandlungsvorrichtung vorzuschlagen. Ferner soll eine Blutbehandlungsvorrichtung zum Verwenden der Flüssigkeitskassette angegeben werden.

Die Aufgabe kann gelöst werden durch eine Flüssigkeitskassette mit den Merkmalen des Anspruchs 1, ferner durch eine Blutbehandlungsvorrichtung mit den Merkmalen des Anspruchs 10.

Obwohl im Folgenden vornehmlich von einer Blutkassette die Rede ist, stellt diese nur eine mögliche erfindungsgemäße Ausgestaltung der vorliegenden Erfindung dar. Wann immer hierin von der Blutkassette die Rede ist, stellt dies keine Beschränkung dar sondern gilt unverändert auch für eine (ebenfalls erfindungsgemäße Flüssigkeitskassette), selbst wenn diese nicht zur Behandlung von Blut verwendet wird.

Erfindungsgemäß wird somit eine Flüssigkeitskassette, insbesondere Blutbehandlungskassette, vorgeschlagen, mit einem als Hartteil ausgestalteten Kassettenkörper. Die Blutbehandlungskassette kann optional eine Folie aufweisen. Ist eine Folie vorgesehen, so ist sie mit dem Hartteil verbunden, deckt das Hartteil zumindest abschnittsweise ab. Das Hartteil weist wenigstens eine erste Zentriervorrichtung und eine zweite Zentriervorrichtung auf. Sie sind bevorzugt an gegenüberliegenden ersten und zweiten Seiten der Blutbehandlungskassette angeordnet oder diesen Seiten zugeordnet.

Ferner wird eine Blutbehandlungsvorrichtung vorgeschlagen, konfiguriert zur Verbindung mit einer erfindungsgemäßen Blutbehandlungskassette, welche eine Aktor-Sensor-Platte mit wenigstens einem Aktuator aufweist. Letzterer ist für eine Interaktion zwischen Aktoren und/oder Sensoren der Blutbehandlungsvorrichtung mit Vorrichtungen der Blutbehandlungskassette konfiguriert.

Bei allen folgenden Ausführungen ist der Gebrauch des Ausdrucks "kann sein" bzw. "kann haben" usw. synonym zu "ist vorzugsweise" bzw. "hat vorzugsweise" usw. zu verstehen und soll eine erfindungsgemäße Ausführungsform erläutern.
Wann immer hierin Zahlenworte genannt werden, so versteht der Fachmann diese als Angabe einer zahlenmäßig unteren Grenze. Sofern dies zu keinem für den Fachmann erkennbaren Widerspruch führt, liest der Fachmann daher beispielsweise bei der Angabe "ein" oder "einem" stets "wenigstens ein" oder "wenigstens einem" mit. Dieses Verständnis ist ebenso von der vorliegenden Erfindung mit umfasst wie die Auslegung, dass ein Zahlenwort wie beispielsweise "ein" alternativ als "genau ein" gemeint sein kann, wo immer dies für den Fachmann erkennbar technisch möglich ist. Beides ist von der vorliegenden Erfindung umfasst und gilt für alle hierin verwendeten Zahlenworte.

Hierin gemachte räumliche Angaben wie "oben", "unten", usw. beziehen sich im Zweifel auf die Darstellung, wie sie den hier beigefügten Figuren zu entnehmen ist.

Vorteilhafte Weiterentwicklungen der vorliegenden Erfindung sind jeweils Gegenstand von Unteransprüchen und Ausführungsformen.

Erfindungsgemäße Ausführungsformen können eines oder mehrere der oben und/oder im Folgenden genannten Merkmale in beliebiger Kombination aufweisen.

In bestimmten beispielhaften erfindungsgemäßen Ausführungsformen weist die Blutbehandlungskassette an einer Vorder- und/oder Rückseite hiervon wenigstens einen, vorzugsweise umlaufenden, Randsteg mit einer Stirnfläche auf. Die Stirnfläche liegt in einer Ebene, welche vorzugsweise parallel zu einer Haupterstreckungsebene der Blutbehandlungskassette liegt.

Die Stirnfläche ist in einigen beispielhaften erfindungsgemäßen Ausführungsformen vorzugsweise eben. Sie liegt in diesen Ausführungsformen also ausschließlich in einer Ebene.

Die Haupterstreckungsebene ist in einigen beispielhaften erfindungsgemäßen Ausführungsformen parallel zu einer von der, falls vorhanden, Folie aufgespannten Ebene oder Haupterstreckungsebene.

In manchen beispielhaften erfindungsgemäßen Ausführungsformen ist die Blutbehandlungskassette ausgestaltet, um im bestimmungsgemäßen Gebrauch der Blutbehandlungskassette in der Haupterstreckungsebene translatorisch spielfrei mit einem ersten Zentrierrastpin einer Blutbehandlungsvorrichtung verbunden zu sein. Die zweite Zentriervorrichtung ist in diesem Ausführungsformen ausgestaltet, um im bestimmungsgemäßen Gebrauch der Blutbehandlungskassette um eine Achse, die vorzugsweise senkrecht oder im Wesentlichen senkrecht zur Haupterstreckungsebene ist, rotatorisch spielfrei mit einem zweiten Zentrierrastpin der Blutbehandlungsvorrichtung verbunden zu sein.

In bestimmten beispielhaften erfindungsgemäßen Ausführungsformen weist die erste Zentriervorrichtung Zentrierfacetten auf, deren Zentrierflächen sich zumindest abschnittsweise jeweils in einer Ebene vorzugsweise senkrecht oder im Wesentlichen senkrecht zur Haupterstreckungsebene der Blutbehandlungskassette erstrecken. In einigen erfindungsgemäßen Ausführungsformen berühren die Zentrierfacetten den ersten Zentrierrastpin zumindest abschnittsweise und vorzugsweise tangentiell.

In manchen beispielhaften erfindungsgemäßen Ausführungsformen weisen die Zentrierfacetten - ganz oder zumindest abschnittsweise - sich in ein Inneres einer Durchlassbohrung oder -öffnung der ersten Zentriervorrichtung hinein wölbende Kanten auf oder sind als solche ausgestaltet.

In bestimmten beispielhaften erfindungsgemäßen Ausführungsformen ist eine Zentrierfacette zumindest teilweise ein Abschnitt eines Einführabschnitts, einer Einführschräge oder eines Einführtrichters einer Zentriervorrichtung, wobei der Einführabschnitt, die Einführschräge oder der Einführtrichter zur Aufnahme eines Abschnitts des Zentrierrastpins dient. Dieser Abschnitt, also die Zentrierfacette selbst, hat - beispielsweise oder vorzugsweise in wenigstens einer Raumrichtung oder in zwei aufeinander senkrecht stehenden Raumrichtungen - eine andere Krümmung und/oder Neigung als andere Abschnitte des Einführabschnitts, der Einführschräge oder des Einführtrichters oder als benachbarte, beispielsweise auf derselben Höhe (etwa bezogen auf eine Einführrichtung oder -tiefe) liegende, Abschnitte des Einführabschnitts, der Einführschräge oder des Einführtrichters.

In einigen beispielhaften erfindungsgemäßen Ausführungsformen ist eine Zentrierfacette ein Abschnitt des Einführtrichters oder des Einführabschnitts, in welchem der Abschnitt oder der Trichter einen größeren Innendurchmesser oder eine größere lichte Weite hat als an anderen und/oder an zur Zentrierfacette benachbarten Abschnitten des Abschnitts oder Trichters.

Der Einführtrichter oder Einführabschnitt weist in manchen beispielhaften erfindungsgemäßen Ausführungsformen, beispielsweise in einer Schnittdarstellung hiervon, zumindest abschnittsweise eine mehr oder weniger ausgeprägte Trichterform auf. Dabei öffnet sich der Einführtrichter zu einem Äußeren der Zentriervorrichtung hin und/oder wird in Einführrichtung des Zentrierrastpins enger.

Der Einführtrichter, der Einführabschnitt oder die Einführschräge können einen nach innen und/oder nach außen (bezogen auf den Durchmesser des Einführtrichters oder des Einführabschnitts, jeweils im Bereich des Einlasses) abgerundeten Einlass oder Rand haben. Die Abrundung kann umlaufend sein oder nur in Abschnitten des Rands vorliegen.

Eine Rastkante ist in manchen beispielhaften erfindungsgemäßen Ausführungsformen als eine Kontaktfläche ähnlich einer Beißfläche einer Beißzange ausgestaltet.

In bestimmten beispielhaften erfindungsgemäßen Ausführungsformen weist die erste Zentriervorrichtung wenigstens einen Abhebel-Randsteg auf. Dieser kann am - bezogen auf die Mitte der Blutbehandlungskassette - äußersten Rand der ersten Zentriervorrichtung liegen oder diese nach lateral begrenzen.

Erfindungsgemäß weisen die erste Zentriervorrichtung und/oder die zweite Zentriervorrichtung jeweils wenigstens eine aktor-sensor-seitige Einführschräge oder wenigstens einen Einführtrichter, zum Aufnehmen eines Abschnitts eines Zentrierrastpins, auf.

In bestimmten beispielhaften erfindungsgemäßen Ausführungsformen weisen die erste und/oder die zweite Zentriervorrichtung wenigstens zwei Rastzungen mit wenigstens je einer Rastkante auf. Die Rastkante verläuft dabei - vorzugsweise vollständig, im Wesentlichen oder zumindest abschnittsweise - in einer Ebene parallel zur Haupterstreckungsebene der Blutbehandlungskassette.

Erfindungsgemäß weist die Durchlassbohrung oder -öffnung der zweiten Zentriervorrichtung im Wesentlichen oder vollständig die Form eines Rechtecks oder eines Langlochs auf.

In bestimmten beispielhaften erfindungsgemäßen Ausführungsformen ist die Längsachse der zweiten Zentriervorrichtung in Richtung auf den Mittelpunkt der Durchlassbohrung oder -öffnung der ersten Zentriervorrichtung ausgerichtet.

Erfindungsgemäß ist die Blutbehandlungsvorrichtung mit einer erfindungsgemäßen Blutbehandlungskassette verbunden.

Erfindungsgemäß weist ein erster Zentrierrastpin und/oder ein zweiter Zentrierrastpin einen Zentrierdurchmesser auf. Er weist ferner einen Rastdurchmesser auf, welcher geringer ist als der Zentrierdurchmesser.

In einigen beispielhaften erfindungsgemäßen Ausführungsformen geht ein zwischen dem Zentrierdurchmesser und dem einen Rastdurchmesser liegender Umfangsabschnitt des Zentrierrastpins mittels gerundeten Übergangs in den Zentrierdurchmesser und/oder in den Rastdurchmesser über.

In bestimmten beispielhaften erfindungsgemäßen Ausführungsformen weist der erste Zentrierrastpin und/oder der zweite Zentrierrastpin eine ebene Stirnfläche auf.

In manchen beispielhaften erfindungsgemäßen Ausführungsformen weist der erste Zentrierrastpin und/oder der zweite Zentrierrastpin eine gerundete Stirnflächenkante auf. Die Stirnkante ist jene Kante, an welcher die Stirnfläche in die Mantelfläche oder die Umfangsfläche des Zentrierrastpins übergeht.

In bestimmten beispielhaften erfindungsgemäßen Ausführungsformen haben der erste Zentrierrastpin und/oder der zweite Zentrierrastpin eine Zentrierhöhe, welche im Bereich des 0,1 bis 0,5-fachen ihres Zentrierdurchmessers liegt. Die Zentrierhöhe ist dabei die freie Höhe oder die Höhe, mit der sich der Zentrierrastpin - vorzugsweise mit oder nur mit seinem Zentrierdurchmesser, nicht aber mit seiner gesamten Höhe - über das Niveau der ihn umgebenden Aktor-Sensor-Matte erhebt. Der Zentrierdurchmesser ist jener Durchmesser des Zentrierrastpins, welcher die Zentrierfunktion des Zentrierrastpins übernimmt. Der Zentrierdurchmesser kann der breiteste Durchmesser des über die Aktor-Sensor-Matte überstehenden Abschnitts des Zentrierrastpins sein. Er kann der Durchmesser jenes Abschnitts des Zentrierrastpins sein, welcher sich unmittelbar oberhalb der Aktor-Sensor-Matte an diese anschließt.

In manchen beispielhaften erfindungsgemäßen Ausführungsformen dienen die erste Zentriervorrichtung und/oder die zweite Zentriervorrichtung nicht nur dem Zentrieren sondern auch dem Verrasten der Blutbehandlungskassette an der Blutbehandlungsvorrichtung.

In bestimmten beispielhaften erfindungsgemäßen Ausführungsformen liegt die Maschinentür bevorzugt auf türseitigen (also maschinenseitigen) Stützstegen und auf den ebenen Stirnflächen der Rastzungen auf.

In einigen beispielhaften erfindungsgemäßen Ausführungsformen ist wenigstens ein Zentrierraststift symmetrisch und/oder als Zylinder ausgestaltet. Er weist in diesen Ausführungsformen einen Zentrierzylinder auf.

In manchen beispielhaften erfindungsgemäßen Ausführungsformen liegen im verpressten Zustand alle Auflageflächen der Maschinentür, welche die Blutbehandlungskassette berühren, parallel zur Haupterstreckungsebene der Kassette. Dies stellt vorteilhaft eine alleinige Zentrierung der Kassette über die Zentrierrastpins sicher.

In bestimmten beispielhaften erfindungsgemäßen Ausführungsformen sind die Zentrierrastpins ausgestaltet, um in Verbindung mit den flachen und entsprechend großzügig bemessenen Stirnflächen der Blutbehandlungskassette um beide Zentriervorrichtungen herum einen ergonomisch günstigen zulässigen Lateralversatz von vorzugsweise dem 0,5 bis 2,5-fachen des Zentrierdurchmessers zu gewährleisen oder diesen hierauf zu beschränken.

In einigen beispielhaften erfindungsgemäßen Ausführungsformen liegt der anfänglich zulässige Lateralversatz der Blutbehandlungskassette, bevor entsprechende Einführschägen in Eingriff kommen, vorzugsweise beim 0,1- bis 0,2-fachen des Zentrierdurchmessers.

In bestimmten beispielhaften erfindungsgemäßen Ausführungsformen ist einer der Zentrierrastpins in eine der Zentriervorrichtungen - nach Abschluss einer einseitigen Einfädelung - soweit eingefädelt, dass eine oder mehrere der Rastkanten der Zentriervorrichtung an den abgerundeten Stirnflächenkanten des Zentrierrastpins anliegen, während der andere Zentrierrastpin seine Einfädelung noch nicht begonnen oder noch nicht abgeschlossen hat.

In bestimmten erfindungsgemäßen Ausführungsformen ist die Blutbehandlungskassette ein Disposable oder ein Einmalartikel.

In bestimmten erfindungsgemäßen Ausführungsformen sind die Blutbehandlungskassette und/oder die Blutbehandlungsvorrichtung zur Apherese, zur Hämodialyse, zur Hämofiltration, Hämodiafiltration, Hämoultrafiltration und dergleichen konfiguriert.

Einige oder alle erfindungsgemäßen Ausführungsformen können einen oder mehrere der oben oder im Folgenden genannten Vorteile aufweisen.

Zu den erzielbaren Vorteilen kann die spielfreie und reproduzierbare Ausrichtung der Blutbehandlungskassette ungeachtet ihrer Herstelltoleranzen und der Toleranzen der Blutbehandlungsvorrichtung zählen.

Von Vorteil kann ferner sein, dass die Blutbehandlungskassette beim Auf- wie beim Abrüsten an beliebigen Stellen gegriffen werden darf und dass trotz der sich dabei unterscheidenden Verkippungs-Konstellationen jede Phase des Auf- und Abrüstens ordnungsgemäß bzw. wie gewünscht abläuft. Ein fehlerhaftes Auf- oder Abrüsten ist damit bei vielen erfindungsgemäßen Ausführungsformen nicht möglich.

Zum Ein- und Ausschnappen ist nur begrenzter Kraftaufwand beim Aufrüsten oder Abrüsten, beispielsweise weniger als 50 N, erforderlich.

Verkippungsbedingte Materialspannungen der Blutbehandlungskassette können vorteilhaft auf Werte deutlich unterhalb jener Schwelle begrenzt werden, ab welcher es zu Zerstörungen an der Blutbehandlungskassette kommen kann; dies gilt auch für ein ruckartiges Abrüsten.

Erfindungsgemäß kann eine ergonomisch einfache manuelle Auf- und Abrüstung der Blutbehandlungskassette auf die Blutbehandlungsvorrichtung gewährleistet werden bei zugleich spielfreier Zentrierung und einer zerstörungssicheren Anordnung. Unzulässig starke Verkantungen und daraus entstehende Materialspannungen können dabei ausgeschlossen werden. Bei den Auf- und Abrüstvorgängen darf die Bedienperson die Blutbehandlungskassette an beliebigen Stellen greifen und belasten. Auch die Reihenfolge der Ein- und Ausrastungen darf beliebig stattfinden.

Die spielfreie und maximal hohe Zentriergenauigkeit wird vorteilhaft erstens durch das Vorsehen zweier voneinander entfernter Zentriervorrichtungen erreicht, wobei die erste Zentriervorrichtung den Nullpunkt der translatorischen Verschiebung definiert und die zweite Zentriervorrichtung, welche bevorzugt langlochartig ausgestaltet ist, die Winkelausrichtung festlegt und dabei die globalen Abmessungstoleranzen zwischen Blutbehandlungsvorrichtung und Blutbehandlungskassette ausgleicht. Zweitens entsteht die Spielfreiheit durch die lokale Anwendung der Facetten-Zentrierung. Lokal bedeutet hier, dass es um die Toleranzzuordnung der Zentrierdurchmesser der Zentrierrastpins zu den facettierten Zentrierbohrungen oder -öffnungen der Zentriervorrichtungen geht (mit deutlich geringeren Toleranzen als beim Abstand zwischen beiden Zentriervorrichtungen).

Aufgrund der geometrischen Gestaltung mit Punktberührung in Verbindung mit der Nachgiebigkeit des Blutbehandlungskassettenmaterials kann die Spielfreiheit in allen Toleranzlagen erreicht werden ohne Materialzerstörung oder zu große Auf- und Abrüstkräfte hervorzurufen.

Aufgrund der manuellen Betätigung in Verbindung mit den spielfreien (oder optional spielarmen) Anordnungen der Zentriervorrichtungen auf den Zentrierrastpins ergeben sich im Stand der Technik die Möglichkeit der Verkantung und die damit verbundenen Materialspannungen und Steigerungen der Auf- und Abrüstkräfte auf bruchgefährliche Werte. Um dieses Problem erfindungsgemäß auszuschließen oder zu miminieren, wird von folgenden Prinzipien vorteilhaft Gebrauch gemacht: Beim Aufrüsten gewährleisten die erfindungsgemäß festgelegten (Rand-) Abstands-, Höhen- und Durchmesserverhältnisse vorteilhaft, dass alle möglichen Verkippungen zwischen Blutbehandlungskassette und Blutbehandlungsvorrichtung in Winkelbereichen bleiben, die unterhalb der kritischen Verkantungswinkel liegen.

Beim Abrüsten sorgt die gewählte Biegesteifigkeit der Blutbehandlungskassette in Verbindung mit den Abständen der Zentrierraststellen zu den entfernten Randstegen der Blutbehandlungskassette und der Zentrierhöhe der Zentrierrastpins dafür, dass durch den Aushebeleffekt an den Randstegen bei verkippender vorheriger einseitiger Ausrastung der verkantungskritische Winkelbereich nicht erreicht werden kann, sondern unmittelbar zuvor die Ausrastung auch der verbleibenden Zentriervorrichtung erfolgt.

Beim Abrüsten sorgt die gewählte Biegesteifigkeit der Blutbehandlungskassette in Verbindung mit dem geringeren Abstand des Randsteges zur ersten Zentriervorrichtung, der gewählten Zentrierhöhe und der verkippten Anordnung der Zentrierfacetten der zweiten Zentriervorrichtung dafür, dass in Verbindung mit dem verringerten Aushebeleffekt bei verkippender einseitiger Ausrastung in der maximal ungünstigsten, möglichen Verkipprichtung der verkantungskritische Winkelbereich vorteilhaft nicht erreicht werden kann.

Beim Abrüsten sorgt die gewählte Biegesteifigkeit der Blutbehandlungskassette in Verbindung mit dem geringeren Abstand des Randsteges zur zweiten Zentriervorrichtung, der gewählten Zentrierhöhe und der langlochförmigen Gestaltung der Zentrierdurchgangsbohrung oder -öffnung der zweiten Zentriervorrichtung dafür, dass in Verbindung mit dem reduzierten Aushebeleffekt bei verkippender einseitiger Ausrastung in der maximal ungünstigsten, möglichen Verkipprichtung der verkantungskritische Winkelbereich vorteilhaft nicht erreicht werden kann.

Beim Auf- und Abrüsten mit beliebigen möglichen Verkippungen erlaubt der Rastdurchmesser der Zentrierrastpins, welcher kleiner als der Zentrierdurchmesser bestimmt wurde, in Verbindung mit seiner gering gewählten Gesamthöhe im Vergleich zur Zentrierhöhe und zum Abstand zwischen den Zentrierraststellen vorteilhaft einen ausreichend großen verkantungsunkritischen Verkippwinkelbereich.

Beim Auf- und Abrüsten mit beliebigen möglichen Verkippungen auch in andere nicht dargestellte Raumrichtungen erlauben die biegbaren Rastzungen und die Einschnitte in Verbindung mit den genannten Abmessungen der Zentrierrastpins und den gewählten Randsteg-Abständen vorteilhaft einen ausreichend großen verkantungsbelastungs-unkritischen Verkippwinkelbereich.

Eine Maschinentür und eine mit ihr erfolgende Verpressung der Blutbehandlungskassette gegen die Aktor-Sensor-Einheit sind nicht zwingend erforderlich, um die dargestellten erfinderischen Merkmale zu gewährleisten. Bereits die spielfreie Zentrierung und Verrastung auf den Zentrierpins können auch bei Verwendung von Blutbehandlungsvorrichtungen, welche keine Maschinentür haben, für eine zuverlässige Halterung und Positionierung der Blutbehandlungskassette ausreichend sein.

Die vorliegende Erfindung wird im Folgenden anhand der beigefügten Zeichnung, in welcher identische Bezugszeichen gleiche oder ähnliche Bauteile bezeichnen, exemplarisch erläutert. In den zum Teil vereinfachten Figuren gilt:
- **Fig. 1**: zeigt eine erfindungsgemäße Blutbehandlungskassette in perspektivischer Draufsicht auf jene Seite hiervon, welche im Gebrauch an einer Aktor-Sensor-Matte der Blutbehandlungsvorrichtung anliegt;
- **Fig. 1a**: zeigt eine vergrößerte Darstellung eines ersten Ausschnitts der Fig. 1a;
- **Fig. 1b**: zeigt eine vergrößerte Darstellung eines zweiten Ausschnitts der Fig. 1a;
- **Fig. 1c**: zeigt eine Vergrößerung eines Ausschnitts aus Fig. 1 im Schnitt;
- **Fig. 2**: zeigt die Blutbehandlungskassette der Fig. 1 in perspektivischer Draufsicht auf die Türseite, also auf die zu jener Seite, welche in Fig. 1 gezeigt ist, gegenüberliegenden Seite, welche im Gebrauch an einer Maschinentür der Blutbehandlungsvorrichtung anliegt; Fig. 2 ist die Darstellung der Blutbehandlungskassette der Fig. 1 nach deren Drehung um 180° um eine Horizontale der Fig. 1;
- **Fig. 3**: zeigt die Detailansicht G der Fig. 2;
- **Fig. 4**: zeigt die Detailansicht F der Fig. 2;
- **Fig. 5**: zeigt die Aktor-Sensor-Einheit der erfindungsgemäßen Blutbehandlungsvorrichtung in perspektivischer Draufsicht auf die Haupterstreckungsebene der Aktor-Sensor-Einheit;
- **Fig. 6**: zeigt die Detailansicht H aus Fig. 5 in Perspektive;
- **Fig. 7**: zeigt die Detailansicht H aus Fig. 5 im Schnitt;
- **Fig. 8**: zeigt in Draufsicht die Blutbehandlungskassette und Abschnitte der Blutbehandlungsvorrichtung im aufgerüsteten Zustand ohne eine rein optionale Maschinentür; Fig. 8 ist die Darstellung der Blutbehandlungskassette der Fig. 1 nach deren Drehung um 180° um eine Horizontale der Fig. 1;
- **Fig. 9**: zeigt einen Schnitt entlang der Linie H-H der Fig. 8; ergänzt ist gegenüber der Fig. 8 die optionale Maschinentür der Blutbehandlungsvorrichtung;
- **Fig. 10**: zeigt eine erste Zentriervorrichtung der erfindungsgemäßen Blutbehandlungskassette im Detail, entlang der Linie S-S der Fig. 8 im Schnitt;
- **Fig. 11**: zeigt eine zweite Zentriervorrichtung der erfindungsgemäßen Blutbehandlungskassette im Detail, entlang der Linie K-K der Fig. 8 im Schnitt;
- **Fig. 12**: zeigt eine Detailansicht der ersten Zentriervorrichtung entlang der Linie H-H der Fig. 8 im Schnitt;
- **Fig. 13**: zeigt eine Detailansicht der zweiten Zentriervorrichtung entlang der Linie H-H der Fig. 8 im Schnitt;
- **Fig. 14**: zeigt einen ersten Schritt des Aufrüstvorgangs, das Ansetzen der Kassette;
- **Fig. 15**: zeigt den zweiten Schritt des Aufrüstvorgangs, den Beginn der Einfädelung;
- **Fig. 16**: zeigt den dritten Schritt des Aufrüstvorgangs, den einseitigen Abschluss der Einfädelung;
- **Fig. 17**: zeigt den vierten Schritt des Aufrüstvorgangs, den beidseitigen Abschluss der Einfädelungen;
- **Fig. 18**: zeigt den fünften Schritt des Aufrüstvorgangs, den einseitigen Abschluss einer Zentrierverrastung;
- **Fig. 19**: zeigt den sechsten Schritt des Aufrüstvorgangs, den beidseitigen Abschluss der Zentrierverrastungen;
- **Fig. 20**: einen kritischen Moment des Ausrastens an der zweiten Zentriervorrichtung während des Abrüstvorgangs;
- **Fig. 21**: zeigt einen kritischen Moment des Ausrastens an der ersten Zentriervorrichtung während des Abrüstvorgangs;
- **Fig. 22**: zeigt den Ausschnitt O der Fig. 20 im Detail;
- **Fig. 23**: zeigt den Ausschnitt P der Fig. 21 im Detail;
- **Fig. 24**: zeigt in einer Prinzipdarstellung die erste und die zweite Zentriervorrichtung mit rein beispielhafter Bemaßung;
- **Fig. 25**: zeigt in einer Prinzipdarstellung die Verkantung mit fortgelassenen Rastzungen;
- **Fig. 26**: in einer Prinzipdarstellung die erste Zentriervorrichtung in der aufgerüsteten Stellung; und
- **Fig. 27**: zeigt in einer Prinzipdarstellung die zweite Zentriervorrichtung im kritischen Moment des Ausrastens.

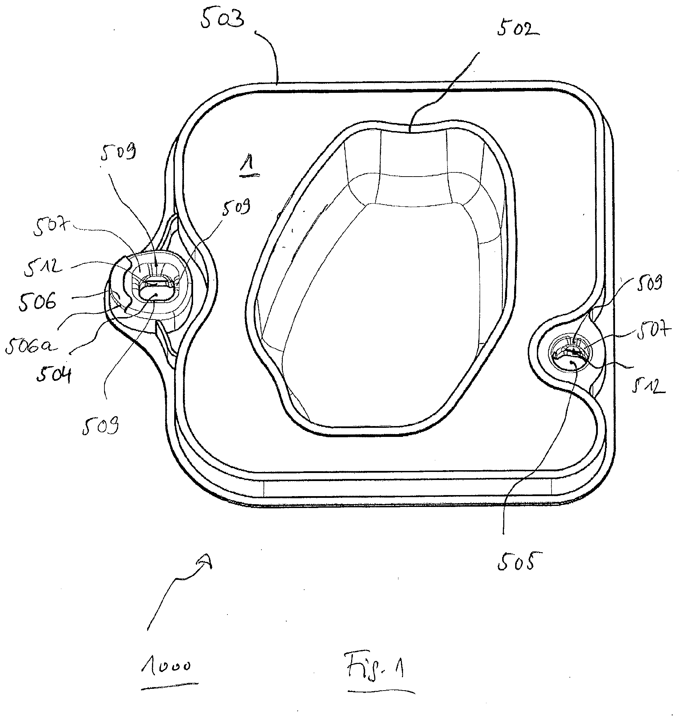

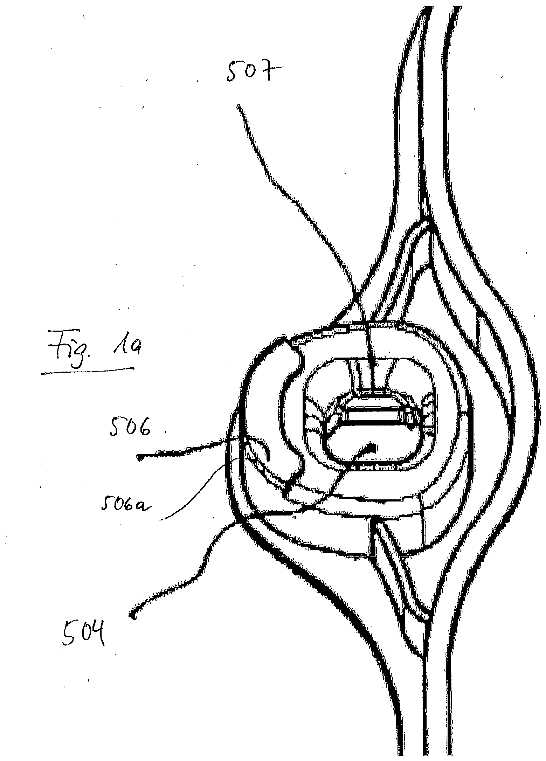

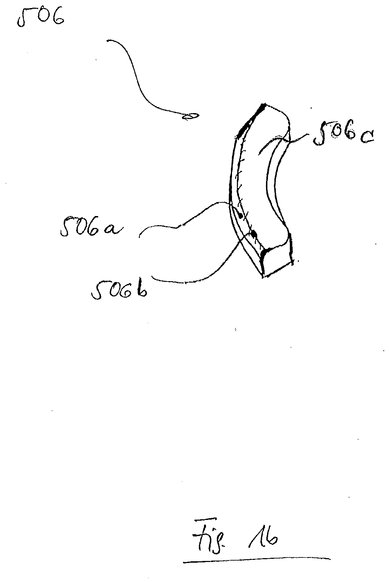

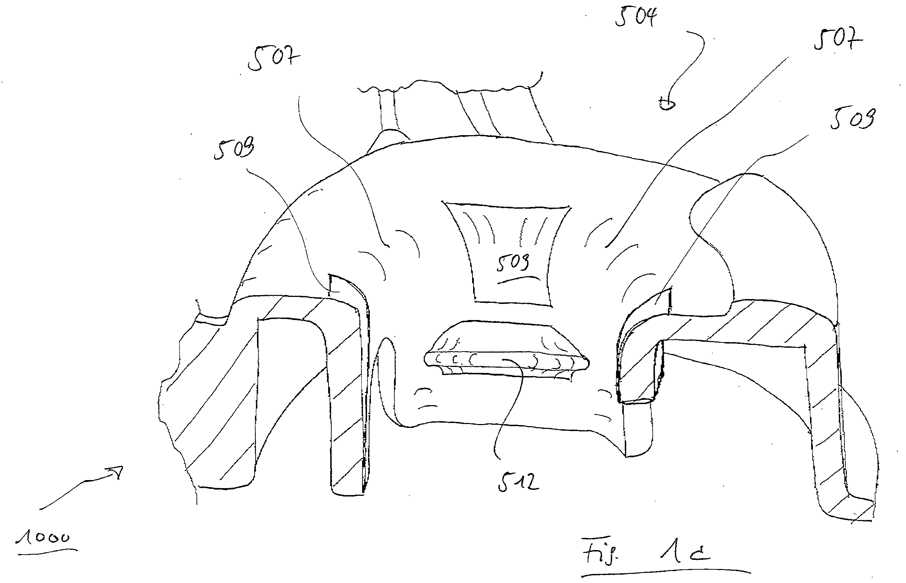

**Fig. 1** zeigt eine erfindungsgemäße Blutbehandlungskassette 1000, hierin auch als Kassette 1000 bezeichnet, in perspektivischer Draufsicht auf deren Haupterstreckungsebene. Gezeigt ist die der Ankopplung der Kassette 1000 mit der in Fig. 1 nicht dargestellten Aktor-Sensor-Einheit 514 der in Fig. 1 ebenfalls nicht dargestellten Blutbehandlungsvorrichtung 5000, hierin auch als Maschine 5000 bezeichnet, dienenden Seite, welche hier als Rückseite oder Ankopplungsseite bezeichnet wird.

Die erfindungsgemäße Blutbehandlungskassette 1000 weist ein Hartteil 1 auf, welches optional im Thermoplast-Spritzguss gefertigt ist. Sein E-Modul-Bereich beträgt bevorzugt zwischen 600 und 2800 N/mm². Im Gebrauch ist das Hartteil 1 vorzugsweise zumindest abschnittsweise mittels einer in Fig. 1 nicht dargestellten Folie 3 bedeckt.

Die Kassette 1000 weist neben Elementen, welche für die Blutbehandlung relevant sind, wie beispielsweise Kammerberandungen 502, wenigstens einen - vorzugsweise umlaufenden - Randsteg 503 auf. Letzterer dient neben der Abdichtung von Abschnitten der Kassette 1000 gegenüber der in Fig. 1 nicht gezeigten Maschine 5000 und der Ausrichtung der Kassette 1000 an der Maschine 5000 mit dem Ziel, zu einer ebenen Anlage der Kassette 1000 an der Maschine 5000 beizutragen, auch als Abhebelsteg bei der Abrüstung der Kassette 1000.

Die Kassette 1000 weist ferner eine erste Zentriervorrichtung 504 auf, welche neben dem Zentrieren der Kassette 1000 bezogen auf die Maschine 5000 auch einem vorübergehenden Befestigen der Kassette 1000 an der Maschine 5000 dienen kann.

Die erste Zentriervorrichtung 504 kann als translatorisch spielfreier Ursprung der Ausrichtung der Kassette 1000 bezeichnet werden. Sie kann, ebenso wie die im Folgenden beschriebene zweite Zentriervorrichtung 505, als Zentrierrasthülse ausgestaltet sein.

Die erste Zentriervorrichtung 504 ist bevorzugt am Rand oder an einer Seite der Kassette 1000 angeordnet. Sie ist bevorzugt in der Mitte einer Seite oder Längsseite, also zentral, angeordnet. Vorzugsweise ist sie unter identischem oder vergleichbarem Abstand jeweils zu ausrichtkritischen, für die Blutbehandlung relevanten Strukturen der Kassette 1000 (z. B. Sensor- und Ventil-Stellen), angeordnet.

Eine zweite Zentriervorrichtung 505 ist als rotatorisch spielfrei ausrichtende Zentrierrasthülse an der Kassette 1000 vorgesehen. Sie liegt vorzugsweise an einer Seite oder Längsseite der Kassette 1000, welche jener Seite, an welcher die erste Zentriervorrichtung 504 liegt, gegenüber liegt. Auf diese Weise wird ein vorteilhaft großer Abstand zwischen der ersten Zentriervorrichtung 504 und der zweiten Zentriervorrichtung 505 erzielt.

Die erste Zentriervorrichtung 504 weist einen Abhebel-Randsteg 506 auf. Sie weist ferner wenigstens eine der Aktor-Sensor-Einheit zugewandte Einführschräge (d. h., dass sich die Einführschräge zur Aktor-Sensor-Einheit hin öffnet, beispielsweise trichterartig öffnet) oder einen solchen Einführtrichter 507 auf.
Mit dem Einführtrichter 507 wird somit in dieser exemplarischen Ausführungsform der verrundete Rand der ersten oder zweiten Zentriervorrichtung 504, 505 bezeichnet. Er dient der groben Vorzentrierung der Kassette 1000.

Der Abhebel-Randsteg 506 ist eine versteifte Auflagefläche (keine Auflagelinie und kein Auflagepunkt sondern -fläche), die beim Verkippen der Kassette beim Abhebeln und Ausrasten die resultierenden Kräfte in die maschinenseitige Aktor-Sensor-Matte 515 einleitet, um die Aktor-Sensor-Matte 515 vor übermäßigem Verschleiß zu bewahren. Der Abhebel-Randsteg 506 kann in der Haupterstreckungsebene vom Rand der Zentriervorrichtung beabstandet sein und/oder erhöht sein, so dass aufgrund des Biegemoments beim Verkippen und Abhebeln und Ausrasten der Kassette eine zum Ausrasten erforderliche, axiale Kraftkomponente auftritt.

Der Abhebel-Randsteg 506 kann, wie in Fig. 1 zu erkennen ist, eine Abschrägungsfacette 506a aufweisen, welche sich an einer seitlichen Fläche des Abhebel-Randstegs 506 befinden kann.

Der Abhebel-Randsteg 506 kann ferner, wie in Fig. 1b zu erkennen ist, eine verrundete Kante 506b aufweisen, welche zwischen der Abschrägungsfacette 506a und einer zumeist horizontalen Stirnfläche 506c des Abhebel-Randstegs 506 liegt.

Der Abhebel-Randsteg 506 geht auch aus dessen vergrößerten Ansichten in **Fig. 1a** und **1b** hervor, wobei Fig. 1a eine Vergrößerung der ersten Zentriervorrichtung 504 in der Ansicht der Fig. 1 zeigt, und wobei Fig. 1b eine Vergrößerung des Abhebel-Randstegs 506 der Fig. 1 oder der Fig. 1a zeigt.

Die erste Zentriervorrichtung 504 und die zweite Zentriervorrichtung 505 weisen in der beispielhaften Ausführungsform der Fig. 1 Zentrierfacetten 509, wie man sie für die erste Zentriervorrichtung 504 besonders gut auch in **Fig. 1c** sehen kann, auf. Dabei ist Fig. 1c eine Vergrößerung eines Ausschnitts aus Fig. 1, welcher für die Darstellung Fig. 1c geschnitten ist, mit Blick von der entgegengesetzten Richtung. Fig. 1c zeigt schnittbedingt nur drei der insgesamt vier über den Umfang des Einführtrichters 507 verteilten Zentrierfacetten 509 der ersten Zentriervorrichtung 504.

Die Zentrierfacetten 509 sind zumindest abschnittsweise oder in ihrem oberen Bereich Teil des Einführtrichters 507. Sie sind dabei oberhalb der Rastkante 512 angeordnet.

Schnittbedingt ist nicht zu sehen, dass hier exemplarisch zwei sich gegenüberliegende Rastkanten 512 vorgesehen sind.

In bestimmten erfindungsgemäßen Ausführungsformen weist die erste Zentriervorrichtung 504 vier Zentrierfacetten 509, die zweite Zentriervorrichtung 505 hingegen nur zwei, wie auch in Fig. 1 zu erkennen ist.

Die Zentrierfacetten 509 können sich in einer Ebene senkrecht zur Haupterstreckungsebene der Kassette 1000 (zumindest lokal oder abschnittsweise) erstrecken.

Die Zentrierfacetten 509 dienen dem lokalen oder punktuellen Kontakt mit dem jeweiligen Zentrierrastpin 517, 518. Sie zentrieren die Zentrierrastpins 517, 518 somit translatorisch oder zentrieren sie jeweils gegenüber einer translatorischen Verschiebung.

Die Zentrierfacetten 509 können beispielsweise direkt an den jeweiligen Einführtrichter 507 angrenzen oder Teil hiervon sein wie oben ausgeführt. Sie erstrecken sich beispielsweise nur um zwischen ca. 5 und 30% des Zentrierdurchmessers 522 in axialer Richtung entlang der Rotationsachse des Zentrierrastpins 517, 518 bzw. der Zentrierrasthülsen oder senkrecht zur Folienebene oder zur Haupterstreckungsebene bevor das Ende der axialen Überlappung mit dem Zentrierbereich (gekennzeichnet durch Zentrierdurchmesser und Zentrierhöhe) des Zentrierrastpins 517, 518) erreicht ist.

In die andere Raumrichtung (also parallel zur Folienebene bzw. senkrecht zur Zentrierrastpin-Symmetrieachse) erstrecken sich die Zentrierfacetten 509 beispielsweise um ca. 10 bis 80 % des Zentrierdurchmessers 522.

Wie in Fig. 1c exemplarisch gezeigt ist, können eine oder alle Zentrierfacetten 509 oben breiter sein als unten.

Zentrierfacetten 509 oder ihre Erstreckungsachsen können in Ebenen senkrecht oder im Wesentlichen senkrecht zur Folienebene bzw. im Wesentlichen parallel zur Zentrierrastpin-Symmetrie-Achse angeordnet sein.

Die Facettenflächen ergeben also kleine Flächen, die jeweils im Wesentlichen Senkrecht zur Folienebene angeordnet sein können. In Kontakt mit dem Zentrierzylinder des Zentrierrastpins 517, 518 entsteht in aufgerüsteter Stellung pro Facette eine Punkt- bis kurze Linienberührung, je nachdem, ob die prismatische Facettenfläche eine Krümmung senkrecht zur Prismenbildungsachse aufweist oder nicht. Um die Verkippungswirkung beim Auf- und Abrüsten kleinstmöglich zu halten, kann die Linienberührung eine Linie sein, die beispielsweise einen Wert im Bereich von 0 bis 30 % des Zentrierdurchmessers 522 beträgt. Diese kurze Berührungslinie erstreckt sich vorzugsweise im Wesentlichen parallel zur Symmetrieachse des Zentrierrastpins 517, 518. Die erste Zentriervorrichtung 504 weist vorzugsweise vier erste Facettenflächen 509 auf (es könnten aber auch drei, fünf, sechs oder mehr Facettenflächen sein). Die zweite Zentriervorrichtung 505 weist im vorliegenden Beispiel zwei (genau zwei, wegen der vorteilhaft bewusst unterbliebenen translatorischen Festlegung entlang der Verbindungsachse zwischen den beiden Zentriervorrichtungen 504, 505) auf.

Bei der zweiten Zentriervorrichtung 505 liegen sich die genau zwei Facettenflächen 509 parallel und spiegelbildlich bezüglich einer Ebene gegenüber, welche im Wesentlichen senkrecht zur Folienebene angeordnet ist, und dabei die Verbindungslinie zwischen den beiden Zentriervorrichtungen 504, 505 sowie beide Symmetrieachsen der beiden Zentrierrastpins 517, 518 enthält.

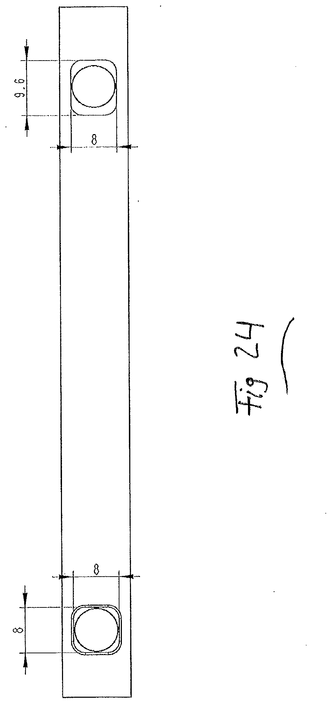

Bei der ersten Zentriervorrichtung 504 liegen sich in einer exemplarischen Ausführungsform je zwei Facettenflächen parallel und spiegelbildlich bezüglich einer Ebene gegenüber, die im Wesentlichen rechtwinklig zur Folienebene angeordnet ist (also die Symmetrieachse der ersten Zentriervorrichtung 504 enthält). Man sieht also in einer Draufsicht auf die Folienebene im Wesentlichen ein an den engsten Stellen quadratisches Zentrier-Loch, siehe Fig. 24. Das Quadrat ist hier zusätzlich wieder ausgerichtet an der Verbindungslinie zwischen den beiden Zentriervorrichtungen. Die Ausrichtung kann derart sein, dass die Verbindungslinie Quadratseiten im rechten Winkel schneidet. Die quadratische Form und diese Ausrichtung müssen aber nicht sein, um die ordnungsgemäße Funktion als translatorisch spielfreier Ausricht-Nullpunkt erfüllen zu können. Vielmehr ist auch die Zentrierloch-Form eines Dreiecks, Fünfecks oder Sechsecks mit beliebiger rotatorischer Ausrichtung möglich und von der vorliegenden Erfindung umfasst.

Die Zentrierfacetten 509 können Teil des Einführtrichters 507 sein. Sie liegen, zumindest abschnittsweise, vorteilhafter Weise in einem oberen Bereich des Einführtrichters 507, siehe auch die vergrößerte Ansicht in Fig. 1c.

In Fig. 1c liegt jene Seite der Kassette 1000, welche im Gebrauch zur Aktor-Sensor-Matte 516 zeigt, oben. Jene Seite der Kassette 1000 bzw. die Seite der Kassette 1000, welche im Gebrauch zur Maschinentür liegt, liegt unten.

Wie in Fig. 1c zu erkennen ist, liegen die Zentrierfacetten 509 in bestimmten beispielhaften Ausführungsformen oberhalb der Rastkante(n) 512. Oberhalb ist hier als näher zur Aktor-Sensor-Matte 516 hin zu verstehen, unterhalb als weiter zur Vorderseite der Kassette hin oder zur Maschinentür hin zu verstehen.

Die Rastkanten 512 sind in bevorzugten, beispielhaften erfindungsgemäßen Ausführungsformen als gerade oder gewölbte Kanten ausgestaltet. Diese Ausgestaltung erlaubt annähernd eine Punkt-Berührung mit einem Rastdurchmesser 521 eines in Fig. 1 nicht gezeigten Zentrierrastpins 517, 518, im Gegensatz zu einer langgestreckten oder (groß-)flächigen Berührung.

Da die erste Zentriervorrichtung 504 und/oder die zweite Zentriervorrichtung 505 in bestimmten beispielhaften erfindungsgemäßen Ausführungsformen nicht nur zentrieren sondern auch für das Verrasten der Kassette 1000 an der Maschine 5000 verantwortlich sind oder hierzu beitragen, können sie in solchen Ausführungsformen auch als Zentrierrastvorrichtungen bezeichnet werden.

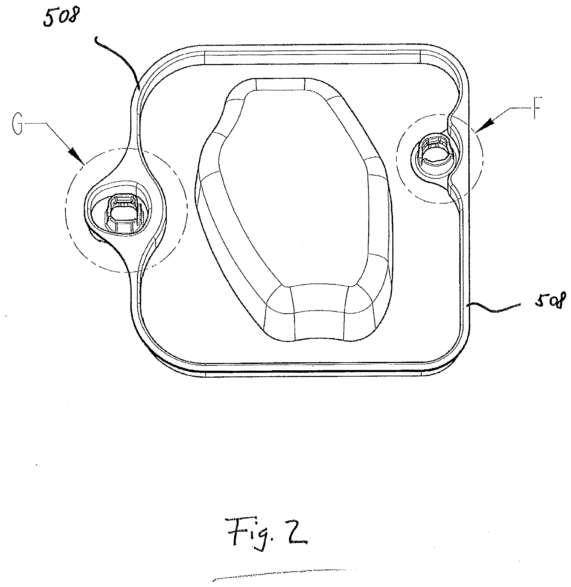

**Fig. 2** zeigt die Kassette 1000 der Fig. 1 in perspektivischer Draufsicht auf die Türseite. Die Türseite kann der optionalen Verpressung mittels einer in Fig. 2 nicht gezeigten Maschinentür 524 dienen. Sie liegt der Ankopplungsseite gegenüber und kann als Vorderseite bezeichnet werden.

Fig. 2 zeigt einen weiteren - hier wiederum nur optional umlaufend gezeigten - Randsteg 508. Dieser kann als multifunktional bezeichnet werden, da er nicht nur als Fläche (bevorzugt in einer Ebene) für die Übertragung der Verpressungswirkung mittels der Maschinentür 524 dient, sondern auch zur Versteifung der Kassette 1000 und zur versteifenden Anbindung der ersten Zentriervorrichtung 504 und/oder der zweiten Zentriervorrichtung 505. Er kann ferner der Übertragung der Auf- und Abrüstkräfte und -wege von jenen (beliebigen) Stellen, an welcher der Benutzer die Kassette 1000 zum Abrüsten anfasst und von welchen ausgehend Kräfte über die erste Zentriervorrichtung 504 und/oder die zweite Zentriervorrichtung 505 in die Kassette 1000 und auf die Zentrierrastpins 517, 518 eingeleitet werden, dienen.

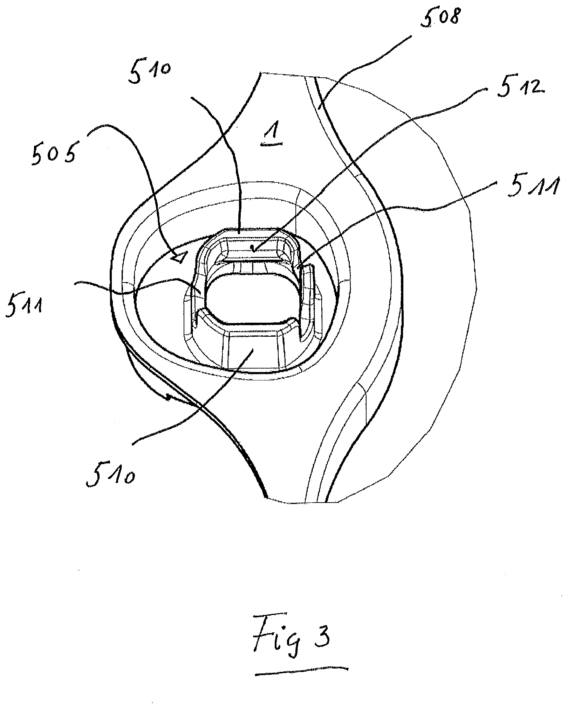

**Fig. 3** zeigt die Detailansicht G der Fig. 2.

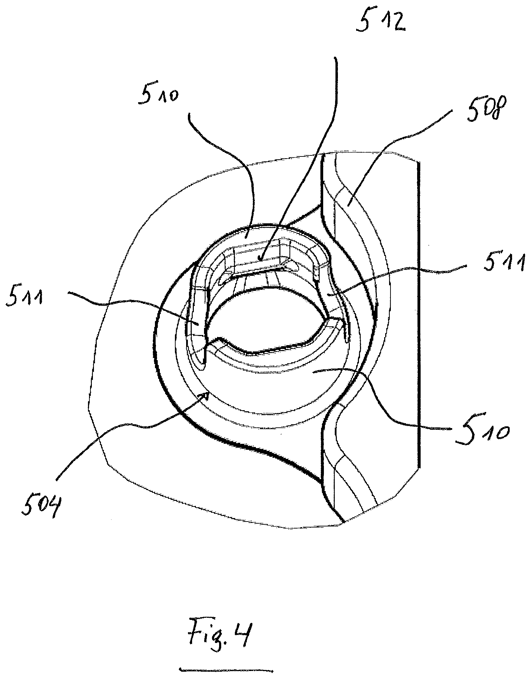

**Fig. 4** zeigt die Detailansicht F der Fig. 2.

Mit dem Bezugszeichen 510 sind in den Figuren Rastzungen bezeichnet, welche Teile der ersten Zentriervorrichtung 504 und/oder der zweiten Zentriervorrichtung 505 sind. Pro Zentriervorrichtung 504, 505 sind vorzugsweise mindestens zwei Rastzungen 510 vorgesehen, vorzugsweise einander gegenüberliegend.

Die Rastzungen 510 können für den bestimmungsgemäßen Gebrauch elastisch biegbar ausgestaltet sein. Sie können durch Einschnitte 511 voneinander getrennt sein, was zur gezielten Biegenachgiebigkeit der Rastzungen 510 beiträgt.

Jede Rastzunge 510 weist wenigstens oder genau eine Rastkante 512 auf. Diese verläuft in einer ersten Ebene parallel zur Haupterstreckungsebene bevorzugt lokal gerade in einer Ebene parallel zur Haupterstreckungsebene der Kassette 1000, um eine Verschiebbarkeit relativ zu den Zentrierrastpins 517, 518 ohne Veränderung der Rast-Haltekraft zu erlauben, und um ferner eine Vorrangigkeit der Zentrierwirkung der Zentrierfacetten 509 zu gewährleisten.

Die Rastkante 512 ist in bevorzugten, erfindungsgemäßen Ausführungsformen in einer zweiten Ebene, welche senkrecht zur Haupterstreckungsebene steht, eine gewölbte Kante oder weist eine solche auf. Diese Form erlaubt dadurch eine Linienberührung mit den ebenfalls gewölbten Eindrehungen der Zentrierrastpins 517, 518 und bewirkt damit Reibungsminimierung bei Auf- und Abrüst-Vorgängen.

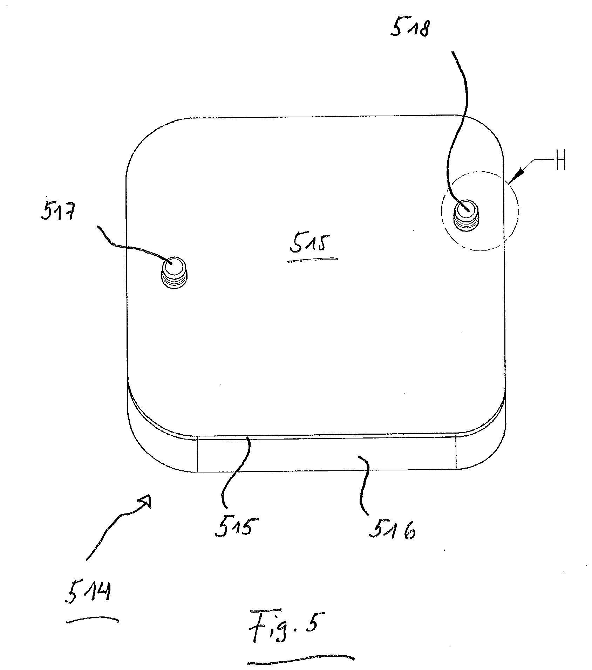

**Fig. 5** zeigt die Aktor-Sensor-Einheit 514 der Maschine 5000 in perspektivischer Draufsicht auf die Haupterstreckungsebene ersterer.

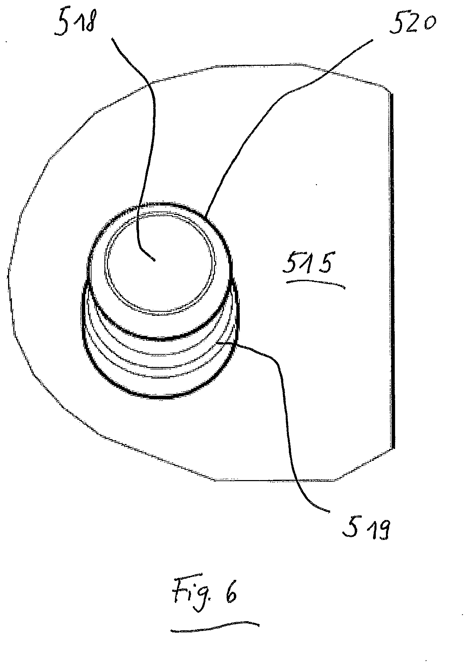

**Fig. 6** ist die Detailansicht H aus Fig. 5 in Perspektive.

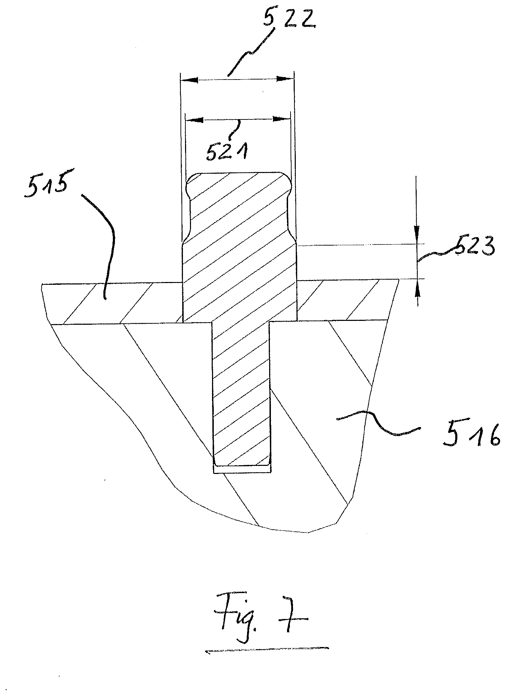

**Fig. 7** ist die Detailansicht H aus Fig. 5 im Schnitt.

Das Bezugszeichen 514 verweist in den Figuren auf die Aktor-Sensor-Einheit 514 der Maschine 5000, wobei 515 die Aktor-Sensor-Matte der Einheit 514 bezeichnet. Die Aktor-Sensor-Matte 515 ist nur optional vorgesehen und zur bevorzugt ebenen Ankopplung an die Kassette 1000 ebenfalls eben ausgestaltet.

516 bezeichnet die Aktor-Sensor-Platte, sie ist steifer Bezugskörper und Verankerungsgrund für den ersten und den zweiten Zentrierrastpin 517 bzw. 518.

Der erste Zentrierrastpin 517 ist zum Verrasten mit der ersten Zentriervorrichtung 504 vorgesehen, während der zweite Zentrierrastpin 518 zum Verrasten mit der zweiten Zentriervorrichtung 505 vorgesehen ist.

Der erste und/oder der zweite Zentrierrastpin 517 bzw. 518 können in bestimmten beispielhaften erfindungsgemäßen Ausführungsformen folgende Eigenschaften aufweisen, welche unabhängig voneinander vorliegen können:
- Sie können als rotationssymmetrische Teile ausgestaltet sein, was kostengünstig, genau und montagegünstig ist.
- Sie können aus steifem, abrasionsbeständigem Material (wie Keramik, Stahl, usw.) gefertigt sein.
- Sie können mit polierter Oberfläche (Reibungsminimierung, Reinigbarkeit, verkantungsmindernd) versehen sein.
- Sie können als identische Elemente ausgestaltet sein (kostengünstig, verwechslungssicher).
- Sie können, wie beispielhaft in Fig. 6 gezeigt, gerundete Übergänge 519 und - vorzugsweise stark - gerundete Stirnflächenkante 520 zur Minimierung der Reibungen bei geraden oder verkanteten Relativbewegungen gegenüber den ersten und zweiten Zentriervorrichtung 504, 505 und zur Optimierung der Einfädelung in die Zentriervorrichtungen 504, 505 haben.
- Sie können einen Rastdurchmesser 521 aufweisen, welcher geringer ist als ihr Zentrierdurchmesser 522. Dies optimiert das Einfädeln und senkt die Gefahr der Verkantung beim Auf- und Abrüsten.
- Sie können eine Zentrierhöhe 523 haben, welche im Bereich des 0,1 bis 0,5-fachen des Zentrierdurchmessers 522 liegt. Dies dient vorteilhaft dazu, den maximalen, verkantungsfähigen Kippwinkel beim Auf- und Abrüsten zu begrenzen.

Die Begrenzung der Verkantung kommt in einigen erfindungsgemäßen Ausführungsformen vorteilhaft dadurch zustande, dass während der verkantenden Ausbaubewegung der Verkantungswinkel stetig zunimmt; wegen der gleichzeitig erfolgenden Abhebelung wird parallel zur Zunahme des Verkantungswinkel die Zentriervorrichtung relativ zum Zentrierrastpin verschoben. Da die Zentrierhöhe 523 des Zentrierrastpins 517, 518 begrenzt ist, kommt die entsprechende Zentriervorrichtung 504, 505 bereits in einem hinsichtlich der Verkantung unkritischen Bereich mit geringem Verkantungswinkel im Bereich der Zentrierfacetten 509 außer Eingriff vom Zentrierdurchmesser 522 der Zentrierrastpins 517, 518.

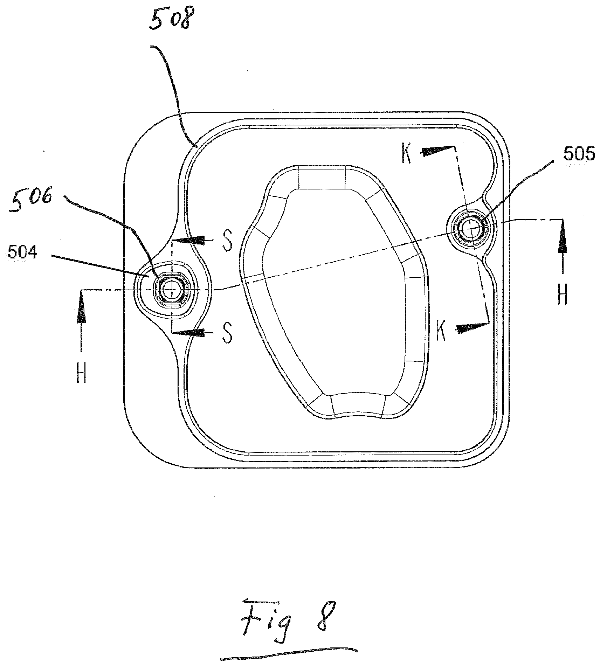

**Fig. 8** zeigt in Draufsicht die Kassette und Abschnitte der Maschine im aufgerüsteten Zustand ohne eine ohnehin rein optionale Maschinentür.

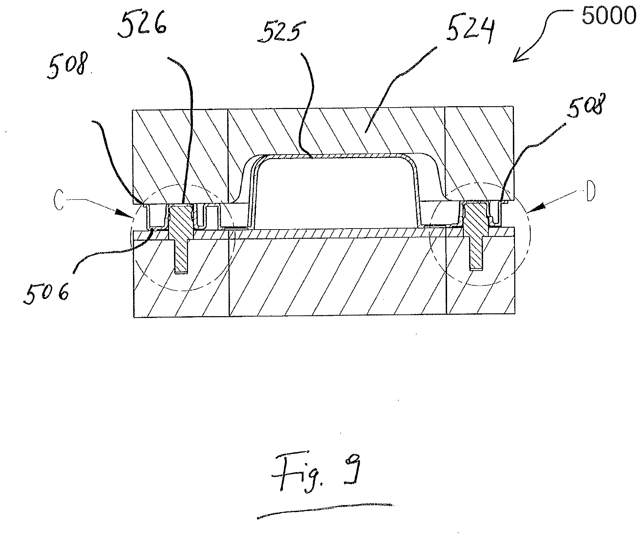

**Fig. 9** zeigt einen Schnitt entlang der Linie H-H der Fig. 8; ergänzt ist gegenüber der Fig. 8 die optionale Maschinentür 524; dargestellt ist der verpresste Zustand.

Die optionale Maschinentür 524 dient dem Verpressen der Kassette 1000 und deren Ausrichtung in einer Ebene, sollte die Kassette 1000 einmal verwunden oder durchgebogen eingelegt worden sein. Das Ausrichten erfolgt an der Aktor-Sensor-Einheit 514 und mittels des umlaufenden Randstegs 508 und ggf. weiterer, optionaler, ebener Auflagezonen 525 und 526.

Die Kassette 1000 liegt während der Blutbehandlung mittels Reibschluss an der Aktor-Sensor-Matte 515 an. Dies stellt sicher, dass die Zentrierposition der Kassette 1000 auch dann beibehalten wird, wenn mechanische oder fluidische Kräfte an der Kassette 1000 oder hiermit verbundenen Verbindungsschläuchen angreifen.

In bestimmten beispielhaften erfindungsgemäßen Ausführungsformen liegen im verpressten Zustand alle Auflageflächen der Maschinentür 524, welche die Kassette 1000 berühren, parallel zur Haupterstreckungsebene der Kassette 1000. Dies stellt vorteilhaft eine alleinige Zentrierung der Kassette 1000 über die Zentrierrastpins 517 und 518 sicher.

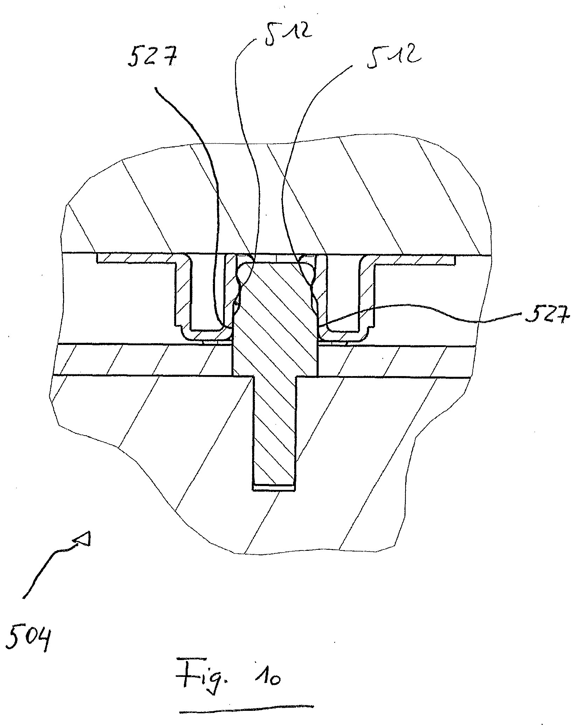

**Fig. 10** zeigt die erste Zentriervorrichtung 504 im Detail entlang der Linie S-S der Fig. 8 im Schnitt.

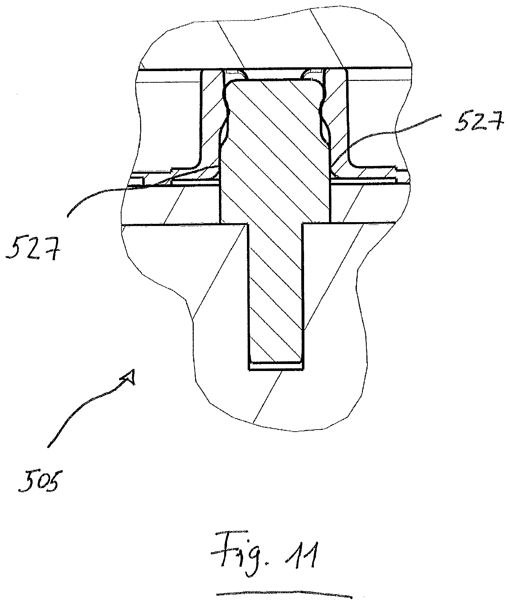

**Fig. 11** zeigt die zweite Zentriervorrichtung 505 im Detail entlang der Linie K-K der Fig. 8 im Schnitt.

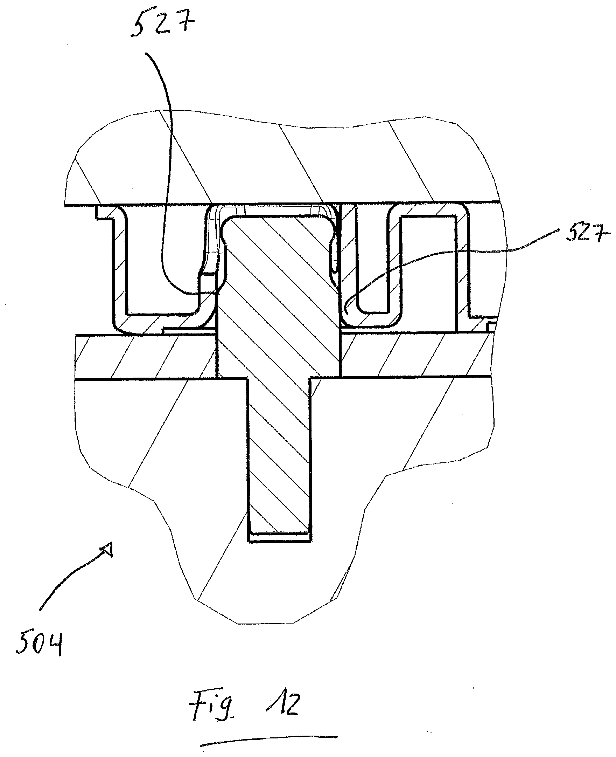

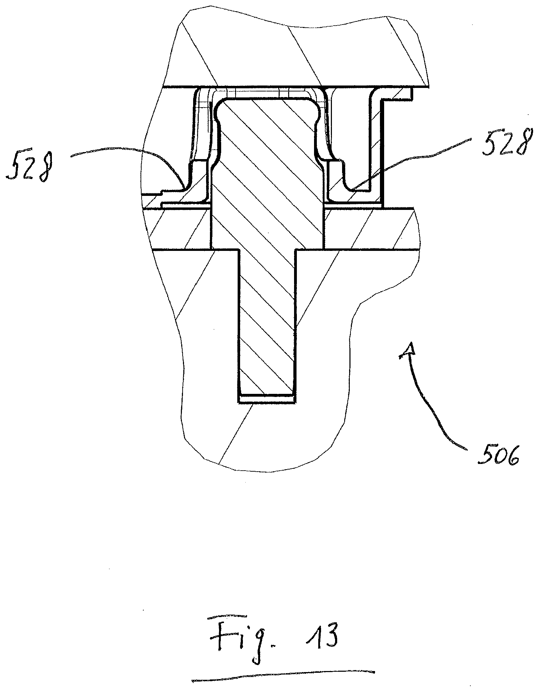

**Fig. 12** und **Fig. 13** zeigen Detailansichten der ersten bzw. zweiten Zentriervorrichtung 504 und 505 entlang der Linie H-H der Fig. 8 im Schnitt.

In jeder der Fig. 10 bis 12 sind die erste und die zweite Zentriervorrichtung 504, 505 mittels der Zentrierrastpins 517 bzw. 518 verrastet.

Fig. 10 zeigt die erste Zentriervorrichtung 504 in einer Schnittrichtung S-S aus Fig. 8, bei der die beiden kurzen gegenüberliegenden Berührlinien oder Linienberührungsabschnitte 527 jeweils gleich lang sind und vergleichsweise lang ausfallen (da der Aushebel-Randabstand zu den Kassetten-Randstegen oben und unten sehr lang ausfällt).

Fig. 11 zeigt einen zum Schnitt der Fig. 10 analogen Schnitt. Das zu Fig. 10 Gesagte kann auch zu Fig. 11 zutreffen.

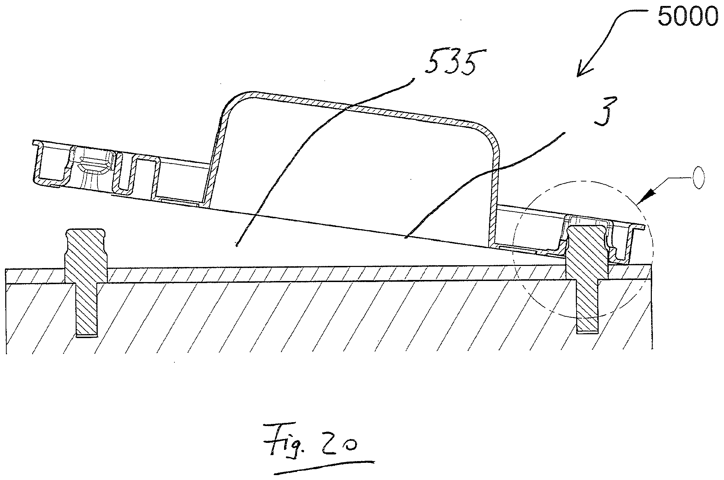

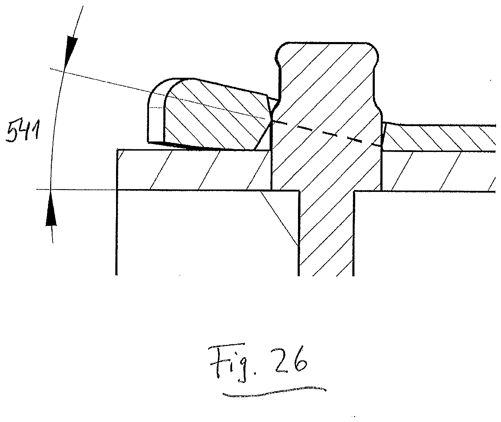

Fig. 12 zeigt den Schnitt durch die erste Zentriervorrichtung entlang des Schnittes H-H, der beim Auf- wie Abrüsten besonders ungünstig von der Einleitung von größeren Verkippungen betroffen ist, wie in den Fig. 20 bis 23 dargestellt. In Fig. 12 sieht man einen linken, kürzeren Linienberührungsabschnitt 527 links, die durch die invertierend vorweggenommene vorverkippte Anordnung der Zentrierengstelle gemäß Fig. 26 zudem weiter entfernt von der Aktor-Sensor-Matte 515 angeordnet ist als der rechte längere Linienberührungsabschnitt 527 rechts.

In den Fig. 10 bis 12 berühren die Linienberührungsabschnitte 527 oder Linienstücke vorzugsweise punktförmig die Zentrierrastpins 517, 518 am Zentrierdurchmesser 522, siehe auch Fig. 24.

Die Toleranzsituation der Durchlassweite zwischen den Linienberührungsabschnitten 527 und den Zentrierdurchmessern 522 der Zentrierrastpins 517, 518 ist vorzugsweise so gewählt, dass stets Spielfreiheit (also kein Spiel) gegeben ist, und zwar ab der spielfreien Anlage bis abgeschlossenen Zentrierverrastung der Kassette 1000. Aufgrund der punktförmigen oder kurzlinigen Anlage aller (hier: vier) Zentrierfacetten 509 der ersten Zentriervorrichtung und der (hier: zwei) Zentrierfacetten 509 der zweiten Zentriervorrichtung. Wird der vergleichweise härtere Zentrierrastpin 517, 518 mit den vergleichweise weicheren Zentrierfacetten 509 verpresst, wobei der Durchmesser des Zentrierrastpins 517, 518 gemessen am Abstand zwischen gegenüberliegenden Zentrierfacetten vorzugsweise ein Übermaß aufweist, verformt sich die weichere Fläche zumindest teilweise elastisch und teilweise plastisch. Bei dieser Umformung wird aus einer Punktberührung eine kreisrunde oder ovale Berührungsfläche und aus einer Linienberührung eine Berührungsfläche. Die Spielfreiheit im verrasteten Zustand bleibt dabei einerseits stets gewährleistet. Die Verpressungskräfte und damit die Reibungskräfte beim Abrüsten sind andererseits aufgrund des niedrigen E-Moduls des Thermoplasten (beispielsweise 1800 N/mm2) und aufgrund der teilweise plastischen Verformung hinreichend gering, so dass es beim Verkanten nicht zum Werkstoffbruch kommt.

Der plastische Anteil der Aufweitung baut sich im Laufe der Behandlung weiter aus, so dass die Abrüstung erleichtert wird. Mittels der Maschinentür 524, welche Reibschluss gewährt, wird eine Verschiebung der Kassette 1000 während der Behandlung zusätzlich sicher verhindert.

Freistellungen bzw. Spalten 528 der zweiten Zentriervorrichtung 505, an welchen Spiel herrscht, verhindern eine Zentrierung in deren Richtung.

Die Längsachse der zweiten Zentriervorrichtung 505 ist in Richtung des Mittelpunkts der Durchlassbohrung oder -öffnung für die erste Zentriervorrichtung 504 ausgerichtet, um die Drehmöglichkeit der Kassette 1000 maximal einzuschränken. Zugleich weist die Längsachse annähernd in die Richtung der kürzesten Entfernung zum Randsteg 508 der Kassette 1000.

Die Einschnitte oder Schlitze 511 zwischen den Rastzungen 510 sind vorzugsweise ebenfalls symmetrisch zur Verbindungsachse oder -linie zwischen der ersten und der zweiten Zentriervorrichtung 504, 505 angeordnet und dienen der Steigerung der Verkipp-Bewegungsfreiheit.

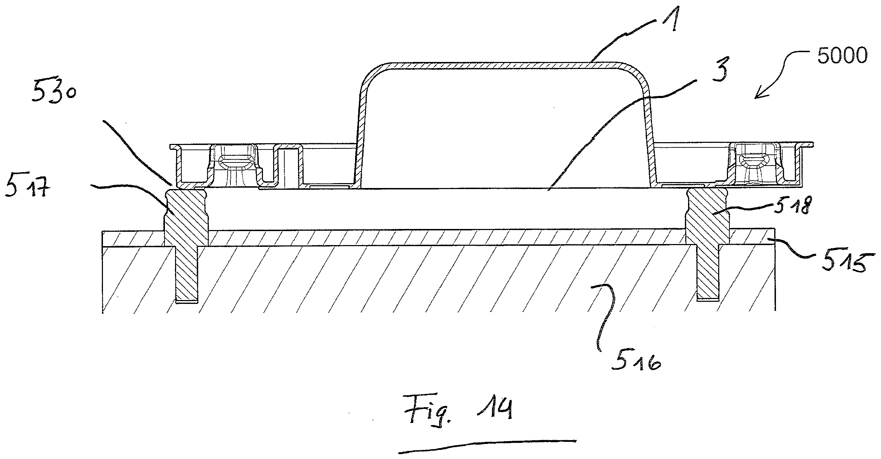

**Fig. 14** zeigt einen ersten Schritt des Aufrüstvorgangs, das Ansetzen der Kassette 1000. Das Bezugszeichen 3 bezeichnet die Folie.

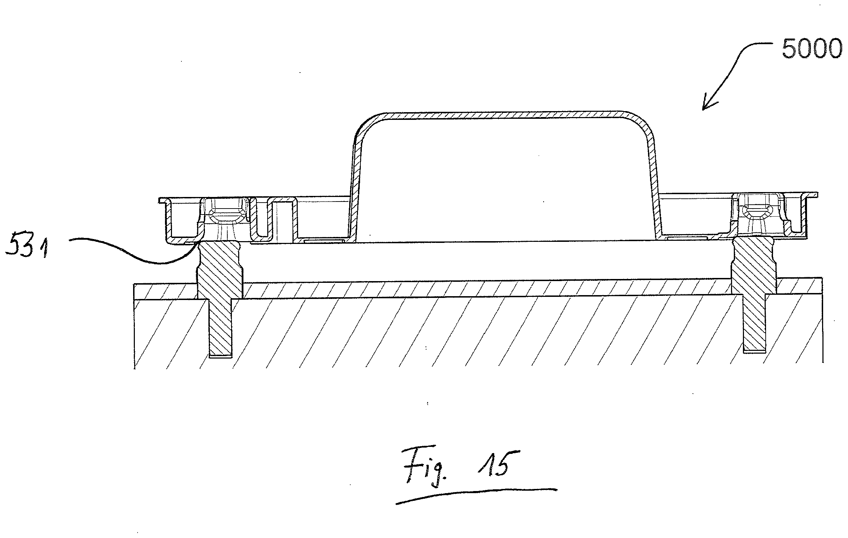

**Fig. 15** zeigt den zweiten Schritt des Aufrüstvorgangs, den Beginn der Einfädelung.

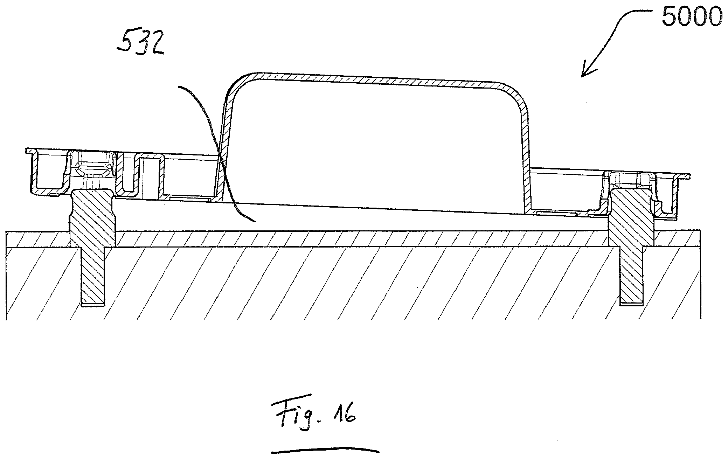

**Fig. 16** zeigt den dritten Schritt des Aufrüstvorgangs, den einseitigen Abschluss der Einfädelung.

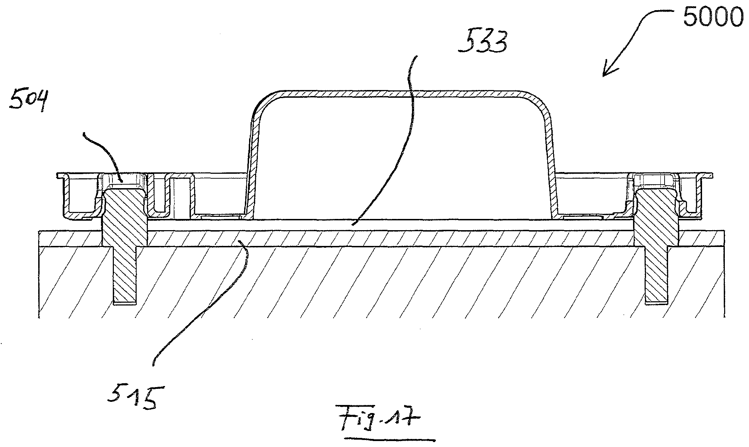

**Fig. 17** zeigt den vierten Schritt des Aufrüstvorgangs, den beidseitigen Abschluss der Einfädelungen.

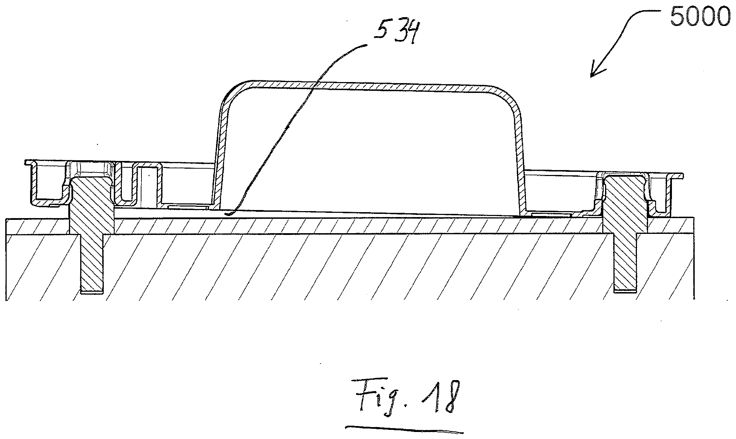

**Fig. 18** zeigt den fünften Schritt des Aufrüstvorgangs, den einseitigen Abschluss einer Zentrierverrastung.

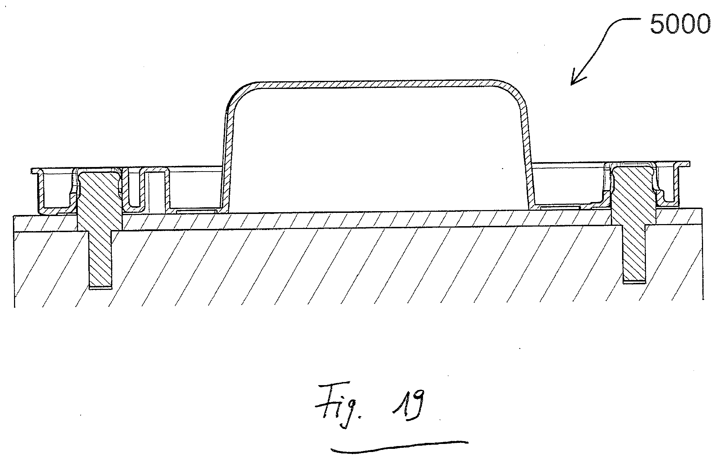

**Fig. 19** zeigt den sechsten Schritt des Aufrüstvorgangs, den beidseitigen Abschluss der Zentierverrastungen.

Bei dem in den vorangegangenen Figuren dargestellten und im Folgenden weiter erläuterten **Aufrüstvorgang** erlauben die flachen Stirnflächen der Zentrierrastpins 517, 518 in Verbindung mit den flachen und entsprechend großzügig bemessenen flachen Stirnflächen der Kassette 1000 um beide Zentriervorrichtungen 504, 505 herum einen ergonomisch günstigen zulässigen Lateralversatz 530 von vorzugsweise dem 0,5- bis 2,5-fachen des Zentrierdurchmessers 522 beim manuellen Ansetzen der Kassette 1000 ohne Beschädigungsrisiko.

Beim Beginn der Einfädelung liegt der Lateralversatz 531 aufgrund entsprechender Einführschrägen 507 vorzugsweise beim 0,1- bis 0,2-fachen des Zentrierdurchmessers 522; dies ist ergonomisch günstig, da die Bedienperson schon weit vor dem Erreichen der Zentrizität das Einfädeln durch die an den Kanten der Einführschrägen sprunghaft beginnende Annäherungsbewegung in Z-Richtung spürt und der weitere Einfädelvorgang dann von den Einführschrägen 507 geleitet wird.

Nach Abschluss einer einseitigen Einfädelung ist eine der Zentriervorrichtungen 504, 505 soweit eingefädelt, dass ihre Rastkanten 512 an den abgerundeten Stirnflächenkanten 520 eines Zentrierrastpins 517, 518 anliegen, während die andere der Zentriervorrichtungen 517, 518 ihre Einfädelung noch nicht begonnen oder noch nicht abgeschlossen hat. Aufgrund des gewählten Abstandes zwischen den Zentriervorrichtungen 517, 518 in Verbindung mit dem Höhenunterschied zwischen der Gesamthöhe eines Zentrierrastpins 517, 518 und der Zentrierhöhe 523 beträgt eine maximale Winkelschiefstellung 532 zwischen der Haupterstreckungsebene der Kassette 1000 und der Haupterstreckungsebene der Maschine 5000 zirka 1° - 3°. In Verbindung mit den geometrischen Gestaltungen 'abgerundete Zentrierrastpin-Stirn', Rastdurchmesser 'kleiner als Zentrierdurchmesser' und 'Einführschrägen der Zentriervorrichtungen' werden bei beliebig ausgeführtem Einfädeln Verkantungen und damit Bewegungsbehinderungen oder Materialüberlastungen verhindert.

Zu Beginn einer Verrastung sind beide Zentriervorrichtungen 504, 505 so weit in die Zentrierrastpins 517, 518 eingeschoben, dass ein Abstand 533 der beiden Haupterstreckungsebenen (von Kassette 1000 einerseits und Aktor-Sensor-Matte 515 andererseits) nur mehr das zirka 0,02 bis 0,04-fache des Zentrierstellenabstands beträgt. Bei beliebiger Reihenfolge und Winkellage der nachfolgenden Verrastung kann eine Winkelschiefstellung 534 von mehr als zirka 1,5 Grad nicht erreicht werden. Diese geringe Winkelschiefstellung 534 verursacht in Verbindung mit der Material- und Geometriewahl noch keine Verkantungskräfte, die zur Überlastung des Materials der Kassette 1000 führen. Diese Verkantungskräfte bedeuten zugleich keine Überschreitung der maximal angestrebten Aufrastkraft von zirka 50 N in allen Toleranzlagen der Abmessungen.

**Fig. 20** zeigt einen kritischen Moment des Ausrastens an der zweiten Zentriervorrichtung 505 während des Abrüstvorgangs.

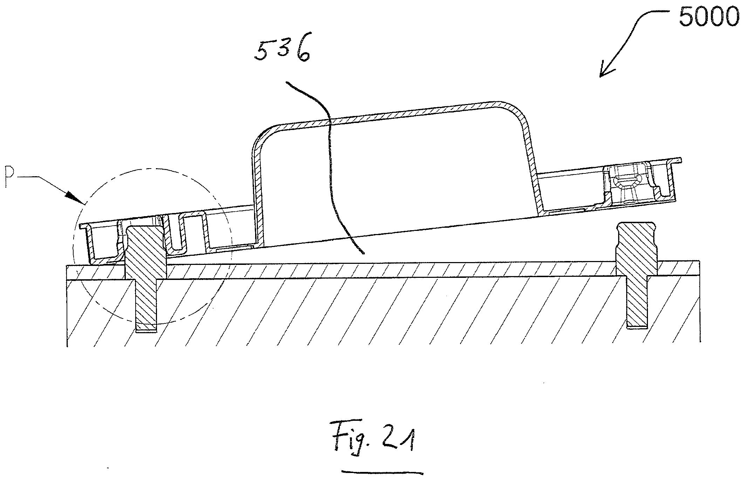

**Fig. 21** zeigt einen kritischen Moment des Ausrastens an der ersten Zentriervorrichtung 504 während des Abrüstvorgangs.

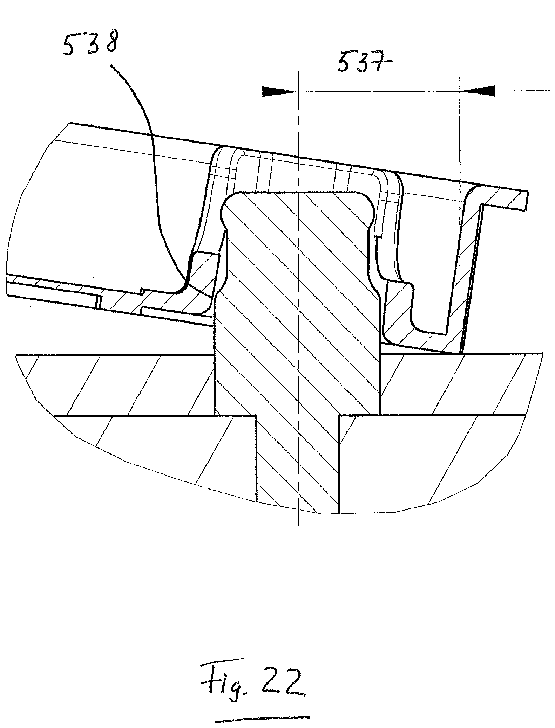

**Fig. 22** zeigt in Detail den Ausschnitt O der Fig. 20.

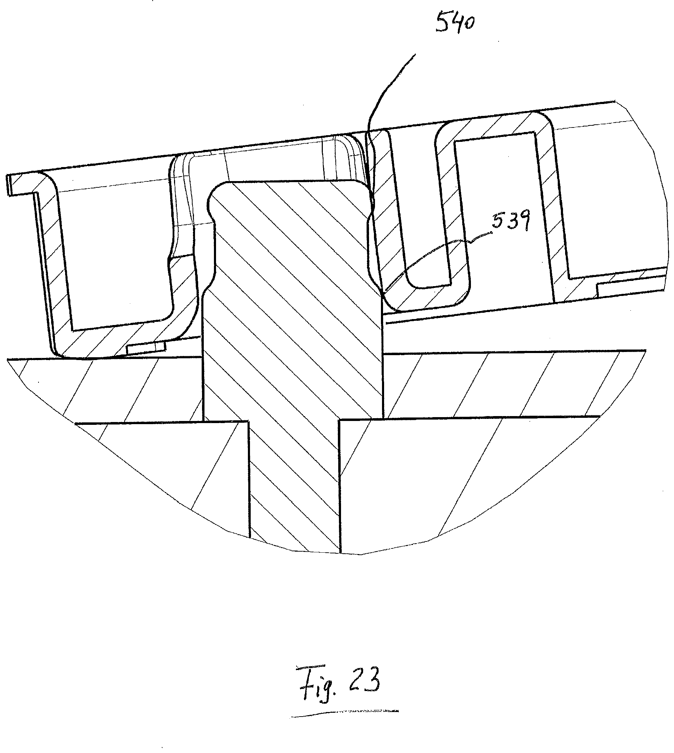

**Fig. 23** zeigt in Detail den Ausschnitt P der Fig. 21.

Bei dem in den vorangegangenen Figuren dargestellten und im Folgenden weiter erläuterten **Abrüstvorgang** findet aufgrund der gewählten Abmessungen beider Zentriervorrichtungen 504, 505 das Ausrasten im Winkelbereich 535, 536 bis zirka maximal 10° statt. Die ungünstigsten Abrüstvorgänge, bei der dieser Kippwinkel im Moment des Ausrastens erreicht werden kann, zeigen die Fig. 20 und 21.

An der Zentriervorrichtung 505 verhindert die langlochartige Durchgangsbohrung oder -öffnung in Verbindung mit den Einschnitten 511 zwischen den Rastzungen 510 und in Verbindung mit einem Aushebel-Randabstand 537 und den Höhen- und Durchmesserverhältnissen das Erreichen der Verkantungssituation 538.

An der ersten Zentriervorrichtung 504 verhindert die verkippte Anordnung 541 der Zentrierdurchgangsbohrung in Verbindung mit den Einschnitten 511 zwischen den Rastzungen 510 und in Verbindung mit dem Aushebel-Randabstand 537 und den Höhen- und Durchmesserverhältnissen das Erreichen der Verkantungssituation 539. Eine gegebenenfalls vorhandene, glatt gestaltete Zunge 540 kann keine kritische Verkantung hervorrufen, da sie wie die Rastzungen 510 biegbar ist.

**Fig. 24** zeigt in einer Prinzipdarstellung die Zentriervorrichtungen 504, 505 mit rein beispielhafter Bemaßung und hierin aufgenommenen Zentrierrastpins.

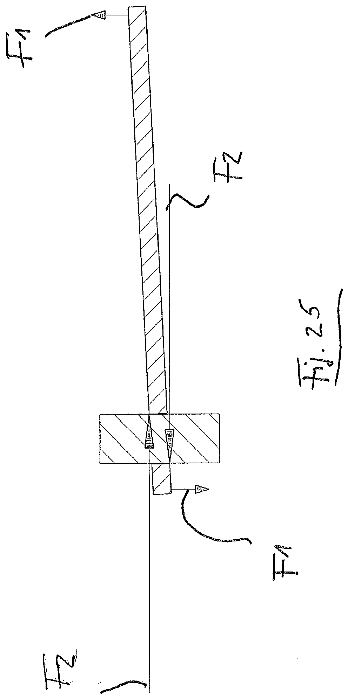

**Fig. 25** zeigt in einer Prinzipdarstellung die Verkantung mit weggelassenen Rastzungen 510.

**Fig. 26** zeigt in einer Prinzipdarstellung die erste Zentriervorrichtung 504 in der aufgerüsteten Stellung.

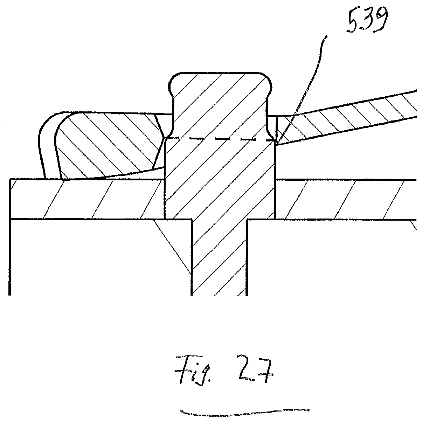

**Fig. 27** zeigt in einer Prinzipdarstellung die zweite Zentriervorrichtung 505 im kritischen Moment des Ausrastens.

In Abhängigkeit vom Aushebel-Randabstand 537 und der Zentrierhöhe 523 beträgt beispielsweise der Anstellwinkel 541 der Durchlassbohrung oder -öffnung der ersten Zentriervorrichtung 504 vorteilhaft ca. 9° bis 14°. Dabei fällt der Moment des Ausrastens der Rastzungen 510 im Wesentlichen mit dem Außer-Eingriff-Bringen 539 der Zentrierfacetten 509 mit dem Zentrierdurchmesser 522 des Zentrierrastpins 517, 518 zusammen, wobei sich der auf die Maschinenebene projizierte Abstand der beiden beteiligten Facetten während der Aushebel-Abrüst-Bewegung kontinuierlich vergrößert und immer größer als der Zentrierdurchmesser 522 bleibt.

### Bezugszeichenliste

- 1000: Blutbehandlungskassette, Flüssigkeitskassette oder Kassette

- 1: Hartteil
- 3: Folie
- 502: Kammerberandungen
- 503: Randsteg
- 504: erste Zentriervorrichtung
- 505: zweite Zentriervorrichtung
- 506: Abhebel-Randsteg
- 506a: Abschrägungsfacette
- 506b: verrundete Kante
- 506c: Stirnfläche
- 507: Einführschräge oder Einführtrichter
- 508: umlaufender Randsteg
- 509: Zentrierfacetten
- 510: Rastzungen
- 511: Einschnitte
- 512: Rastkante
- 514: Aktor-Sensor-Einheit
- 515: Aktor-Sensor-Matte
- 516: Aktor-Sensor-Platte
- 517: erster Zentrierrastpin
- 518: zweiter Zentrierrastpin
- 519: Übergänge
- 520: Stirnflächenkante
- 521: Rastdurchmesser
- 522: Zentrierdurchmesser
- 523: Zentrierhöhe
- 524: Maschinentür
- 525: Auflagezone
- 526: Auflagezone
- 527: Linienberührungsabschnitte
- 528: Freistellung

- 530: Lateralversatz
- 531: Lateralversatz
- 532: maximale Winkelschiefstellung
- 533: Abstand
- 534: Winkelschiefstellung
- 535: Winkelbereich
- 536: Winkelbereich
- 537: Aushebel-Randabstand
- 538: Verkantungssituation
- 539: Verkantungssituation
- 540: Zunge
- 541: verkippte Anordnung

- 5000: Maschine oder Blutbehandlungsvorrichtung

## Patentansprüche

1. Medizinische Flüssigkeitskassette, insbesondere Blutbehandlungskassette (1000), mit einem als Hartteil (1) ausgestalteten Kassettenkörper und optional einer Folie (3), welche mit dem Hartteil (1) verbunden ist und das Hartteil (1) zumindest abschnittsweise abdeckt,
wobei das Hartteil (1) wenigstens eine erste Zentriervorrichtung (504) und eine zweite Zentriervorrichtung (505) aufweist, welche optional an gegenüberliegenden ersten und zweiten Seiten der Flüssigkeitskassette angeordnet oder diesen zugeordnet sind, und
wobei die erste Zentriervorrichtung (504) und/oder die zweite Zentriervorrichtung (505) jeweils wenigstens eine(n) aktor-sensor-seitige Einführschräge oder Einführtrichter (507) zum Aufnehmen eines Abschnitts eines Zentrierrastpins aufweist, und
wobei eine Durchlassbohrung oder -öffnung der zweiten Zentriervorrichtung (505) die Form eines Rechtecks oder eines Langlochs aufweist.

2. Flüssigkeitskassette nach Anspruch 1, welche an einer Vorder- und/oder Rückseite hiervon wenigstens einen, vorzugsweise umlaufenden, Randsteg (503, 508) mit einer Stirnfläche aufweist, wobei die Stirnfläche in einer Ebene liegt, wobei die Ebene vorzugsweise parallel zu einer Haupterstreckungsebene der Flüssigkeitskassette liegt.

3. Flüssigkeitskassette nach Anspruch 1 oder 2, wobei die erste Zentriervorrichtung (504) ausgestaltet ist, um im Gebrauch translatorisch spielfrei mit einem ersten Zentrierrastpin (517) einer Blutbehandlungsvorrichtung (5000) verbunden zu sein, wohingegen die zweite Zentriervorrichtung (505) ausgestaltet ist, um im Gebrauch rotatorisch spielfrei mit einem zweiten Zentrierrastpin (518) der Blutbehandlungsvorrichtung (5000) verbunden zu sein.

4. Flüssigkeitskassette nach einem der vorangegangenen Ansprüche, wobei die erste Zentriervorrichtung (504) Zentrierfacetten (509) aufweist, welche sich - vorzugsweise ganz oder im Wesentlichen - in einer Ebene senkrecht zur Haupterstreckungsebene der Flüssigkeitskassette erstrecken.

5. Flüssigkeitskassette nach Anspruch 4, wobei die Zentrierfacetten (509) ganz oder zumindest abschnittsweise in einem Inneren einer Durchlassbohrung oder -öffnung der ersten Zentriervorrichtung (504) angeordnete Flächen sind oder solche aufweisen.

6. Flüssigkeitskassette nach einem der vorangegangenen Ansprüche, wobei die erste Zentriervorrichtung (504) wenigstens einen Abhebel-Randsteg (506) aufweist.

7. Flüssigkeitskassette nach einem der vorangegangenen Ansprüche, wobei die erste und/oder die zweite Zentriervorrichtung (504, 505) wenigstens je zwei Rastzungen (510) mit wenigstens je einer Rastkante (512) aufweist, wobei die Rastkante (512) in einer Ebene parallel zur Haupterstreckungsebene der Kassette (1000) verläuft.

8. Flüssigkeitskassette nach einem der vorangegangenen Ansprüche, wobei die Längsachse der zweiten Zentriervorrichtung (505) - vorzugsweise ganz oder im Wesentlichen - in Richtung auf den Mittelpunkt der Durchlassbohrung oder -öffnung der ersten Zentriervorrichtung (504) ausgerichtet ist.

9. Flüssigkeitskassette nach Anspruch 7 oder nach Ansprüche 7 und 8, wobei zwischen benachbarten Rastzungen (510) vorhandene Einschnitte oder Schlitze (511) symmetrisch zur Verbindungsachse oder -linie zwischen der ersten und der zweiten Zentriervorrichtung (504, 505) angeordnet sind.

10. Blutbehandlungsvorrichtung (5000), zur Verbindung mit einer Flüssigkeitskassette nach einem der Ansprüche 1 bis 9, mit einer Aktor-Sensor-Platte (516) mit wenigstens einem Aktuator für eine Interaktion zwischen Aktoren und/oder Sensoren der Blutbehandlungsvorrichtung (5000) mit Vorrichtungen der Flüssigkeitskassette, wobei ein erster Zentrierrastpin (517) und/oder ein zweiter Zentrierrastpin (518) einen Zentrierdurchmesser (522) und ferner einen Rastdurchmesser (521) aufweisen, welcher geringer ist als ihr Zentrierdurchmesser (522).

11. Blutbehandlungsvorrichtung (5000) nach Anspruch 10, verbunden mit einer Flüssigkeitskassette nach einem der Ansprüche 1 bis 9.

12. Blutbehandlungsvorrichtung (5000) nach Anspruch 11, wobei ein zwischen dem Zentrierdurchmesser (522) und dem einen Rastdurchmesser (521) liegender Umfangsabschnitt des Zentrierrastpins (517, 518) mittels gerundeten Übergangs (519) in den Zentrierdurchmesser (522) und/oder den Rastdurchmesser (521) übergeht.

13. Blutbehandlungsvorrichtung (5000) nach einem der Ansprüche 10 bis 12, wobei der erste Zentrierrastpin (517) und/oder der zweite Zentrierrastpin (518) eine ebene Stirnfläche (520) aufweist.

14. Blutbehandlungsvorrichtung (5000) nach einem der Ansprüche 10 bis 13, wobei der erste Zentrierrastpin (517) und/oder der zweite Zentrierrastpin (518) eine gerundete Stirnflächenkante (520) aufweist.

15. Blutbehandlungsvorrichtung (5000) nach einem der Ansprüche 10 bis 14, wobei der erste Zentrierrastpin (517) und/oder der zweite Zentrierrastpin (518) eine Zentrierhöhe (523) haben, welche im Bereich des 0,1 bis 0,5-fachen ihres Zentrierdurchmessers (522) liegt.

## Claims

1. A medical liquid cassette, in particular a blood treatment cassette (1000), comprising a cassette body designed as a hard part (1) and optionally a film (3), which is connected to the hard part (1) and at least partially covers the hard part (1),
wherein the hard part (1) comprises at least a first centering apparatus (504) and a second centering apparatus (505), which are optionally arranged on a first and a second opposite sides of the liquid cassette or which are associated with those, and
wherein the first centering apparatus (504) and/or the second centering apparatus (505) each comprises at least one actuator-sensor-side insertion bevel or insertion funnel (507) for receiving a portion of a centering-latching pin, and
wherein an outlet bore or opening of the second centering apparatus (505) has the shape of a rectangle or an oblong hole.

2. The liquid cassette according to claim 1, comprising on its front surface and/or on its rear side at least one, preferably circumferential, edge web (503, 508) with a front surface, the front surface lying in a plane, wherein the plane lies preferably parallel to a main extension plane of the liquid cassette.

3. The liquid cassette according to claim 1 or 2, wherein the first centering apparatus (504) is designed to be connected in use without translational play to a first centering latching pin (517) of a blood treatment apparatus (5000), whereas the second centering apparatus (505) is designed to be connected in use without rotational play to a second centering latching pin (518) of the blood treatment apparatus (5000).

4. The liquid cassette according to anyone of the preceding claims, wherein the first centering apparatus (504) comprises centering facets (509) extending, preferably completely or essentially, in a plane perpendicular to the main extension plane of the liquid cassette.

5. The liquid cassette according to claim 4, wherein the centering facets (509) are surfaces, which are arranged, completely or at least partially, in an interior of an outlet bore or opening of the first centering apparatus (504), or which comprise such ones.

6. The liquid cassette according to anyone of the preceding claims, wherein the first centering apparatus (504) comprises at least one lever peripheral edge (506).

7. The liquid cassette according to anyone of the preceding claims, wherein the first and/or the second centering apparatus (504, 505) comprise(s) each at least two latching tongues (510), each with at least one latching edge (512), wherein the latching edge (512) extends in a plane parallel to the main extension plane of the cassette (1000).

8. The liquid cassette according to anyone of the preceding claims, wherein the longitudinal axis of the second centering apparatus (505) - preferably completely or essentially - is oriented in the direction of the center of the outlet bore or opening of the first centering apparatus (504).

9. The liquid cassette according to claim 7 or claims 7 and 8, wherein existing incisions or slots (511) between adjacent latching tongues (510) are arranged symmetrically with regard to the connection axis or connection line between the first and the second centering apparatus (504, 505).

10. A blood treatment apparatus (5000), for connection to a liquid cassette according to one of claims 1 to 9, comprising an actuator-sensor-plate (516) with at least one actuator for an interaction between actuators and/or sensors of the blood treatment apparatus (5000) with apparatuses of the liquid cassette, wherein a first centering latching pin (517) and/or a second centering latching pin (518) comprise (s) a centering diameter (522) and further a latching diameter (521), which is/are less than its/their centering diameter (522).

11. The blood treatment apparatus (5000) according to claim 10, which is connected to a liquid cassette according to one of claims 1 to 9.

12. The blood treatment apparatus (5000) according to claim 11, wherein a circumferential section of the centering latching pin (517, 518) lying between the centering diameter (522) and the one latching diameter (521) goes over into the centering diameter (522) and/or the latching diameter (521) by means of a rounded passage (519).

13. The blood treatment apparatus (5000) according to anyone of claims 10 to 12, wherein the first centering latching pin (517) and/or the second centering latching pin (518) comprise(s) a flat front surface (520).

14. The blood treatment apparatus (5000) according to anyone of claims 10 to 13, wherein the first centering latching pin (517) and/or the second centering latching pin (518) comprise(s) a rounded front surface edge (520).

15. The blood treatment apparatus (5000) according to anyone of claims 10 to 14, wherein the first centering latching pin (517) and/or the second centering latching pin (518) have/has a centering height (523) lying in the range of 0.1 to 0.5 times its/their centering diameter (522).

## Revendications

1. Une cassette médicale pour liquide, en particulier une cassette pour traitement du sang (1000), comprenant un corps de cassette conçu comme une partie dure (1) et éventuellement un film (3), lequel est relié à la partie dure (1) et recouvre au moins partiellement la partie dure (1),
où la partie dure (1) comprend au moins un premier appareil de centrage (504) et un second appareil de centrage (505), qui sont éventuellement disposés sur un premier et un second côtés opposés de la cassette pour liquide ou associés à ceux-ci, et
où le premier appareil de centrage (504) et/ou le second appareil de centrage (505) comprend/comprennent chacun au moins une rampe d'insertion ou un entonnoir d'insertion (507) côté actionneur-capteur destiné à recevoir une partie d'une broche de verrouillage et de centrage, et
où un alésage ou un orifice de sortie du second appareil de centrage (505) a la forme d'un rectangle ou d'un trou oblong.

2. La cassette pour liquide selon la première revendication, comprenant sur sa face avant et/ou sur sa face arrière au moins un rebord périphérique (503, 508), de préférence circonférentiel, avec une surface frontale, la surface frontale se situant dans un plan, le plan étant de préférence parallèle à un plan principal d'extension de la cassette pour liquide.

3. La cassette pour liquide selon la revendication 1 ou 2, où le premier appareil de centrage (504) est conçu pour être relié lors de l'utilisation sans jeu de translation à une première broche de verrouillage et de centrage (517) d'un appareil de traitement du sang (5000), tandis que le second appareil de centrage (505) est conçu pour être relié lors de l'utilisation sans jeu de rotation à une seconde broche de verrouillage et de centrage (518) de l'appareil de traitement du sang (5000).

4. La cassette pour liquide selon l'une quelconque des revendications précédentes, où le premier appareil de centrage (504) comprend des facettes de centrage (509) s'étendant, de préférence complètement ou essentiellement, dans un plan perpendiculaire au plan principal d'extension de la cassette pour liquide.

5. La cassette pour liquide selon la revendication 4, où les facettes de centrage (509) sont des surfaces agencées, complètement ou au moins partiellement, à l'intérieur d'un alésage ou d'un orifice de sortie du premier appareil de centrage (504), ou comprennent de telles surfaces.

6. La cassette pour liquide selon l'une quelconque des revendications précédentes, où le premier appareil de centrage (504) comprend au moins un rebord périphérique levier (506).

7. La cassette pour liquide selon l'une quelconque des revendications précédentes, où le premier et/ou le second appareil(s) de centrage (504, 505) comprend/comprennent chacun au moins deux languettes de verrouillage (510), ayant chacune au moins un bord d'arrêt (512), le bord d'arrêt (512) s'étendant dans un plan parallèle au plan principal d'extension de la cassette (1000).

8. La cassette pour liquide selon l'une quelconque des revendications précédentes, où l'axe longitudinal du second appareil de centrage (505) est orienté, de préférence complètement ou essentiellement, dans la direction du centre de l'alésage ou de l'orifice de sortie du premier appareil de centrage (504).

9. La cassette pour liquide selon la revendication 7, ou selon les revendications 7 et 8, où des incisions ou des fentes (511) existantes entre des languettes de verrouillage (510) adjacentes sont agencées de façon symétrique par rapport à l'axe ou à la ligne de connexion entre le premier et le second appareil de centrage (504, 505).

10. Un appareil de traitement du sang (5000) destiné à être connecté à une cassette pour liquide selon l'une des revendications 1 à 9, comprenant une plaque capteur-actionneur (516) ayant au moins un actionneur pour permettre une interaction entre des actionneurs et/ou des capteurs de l'appareil de traitement du sang (5000) avec des appareils de la cassette pour liquide, où une première broche de verrouillage et de centrage (517) et/ou une seconde broche de verrouillage et de centrage (518) a/ont un diamètre de centrage (522), et de plus, un diamètre de verrouillage (521), inférieur(s) à son/leur diamètre de centrage (522).

11. L'appareil de traitement du sang (5000) selon la revendication 10, qui est connecté à la cassette pour liquide selon l'une des revendications 1 à 9.

12. L'appareil de traitement du sang (5000) selon la revendication 11, où une section circonférentielle de la broche de verrouillage et de centrage (517, 518) située entre le diamètre de centrage (522) et le diamètre de verrouillage (521) passe dans le diamètre de centrage (522) et/ou dans le diamètre de verrouillage (521) au moyen d'une transition arrondie (519).

13. L'appareil de traitement du sang (5000) selon l'une quelconque des revendications 10 à 12, où la première broche de verrouillage (517) et/ou la seconde broche de verrouillage (518) comprend/comprennent une surface frontale plane (520).

14. L'appareil de traitement du sang (5000) selon l'une quelconque des revendications 10 à 13, où la première broche de verrouillage (517) et/ou la seconde broche de verrouillage (518) comprend/comprennent un bord de surface frontale arrondi (520).

15. L'appareil de traitement du sang (5000) selon l'une quelconque des revendications 10 à 14, où la première broche de verrouillage (517) et/ou la seconde broche de verrouillage (518) a/ont une hauteur de centrage (523) comprise entre 0,1 et 0,5 fois son/leur diamètre de centrage (522).
